# EUROPÄISCHE PATENTSCHRIFT

(11) **EP 1 549 647 B1**
(45) Veröffentlichungstag und Bekanntmachung des Hinweises auf die Patenterteilung: **12.04.2006**
(21) Anmeldenummer: 03748122.3
(22) Anmeldetag: 07.10.2003
(51) Int. Cl.: C07D 471/04, A61K 31/345, C07D 235/00, C07D 221/00

(54) **SUBSTITUIERTE C-IMIDAZO¬1,2-A|PYRIDIN-3-YL-METHYLAMINE**
SUBSTITUTED C-IMIDAZO¬1,2-A|PYRIDIN-3-YL-METHYLAMINES
C-IMIDAZO¬1,2-A|PYRIDIN-3-YL-METHYLAMINES SUBSTITUEES

(30) Priorität: 10.10.2002 DE 10247269
(43) Veröffentlichungstag der Anmeldung: 06.07.2005
(73) Patentinhaber: Grünenthal GmbH, 52078 Aachen (DE)
(72) Erfinder: OBERBÖRSCH, Stefan, 52064 Aachen (DE); SUNDERMANN, Bernd, 52066 Aachen (DE); SUNDERMANN, Corinna, 52066 Aachen (DE); HENNIES, Hagen-Heinrich, 52152 Simmerath (DE)
(86) Internationale Anmeldenummer: PCT/EP2003/011079
(87) Internationale Veröffentlichungsnummer: WO 2004/035578

(56) Entgegenhaltungen:
- EP-A- 0 172 096
- ALMIRANTE: "BOLL. CHIM. FARM" BOLLETTINO CHIMICO FARMACEUTICO, SOCIETA EDITORIALE FARMACEUTICA, MILANO, IT, Bd. 105, 1966, Seiten 32-44, XP001055328 ISSN: 0006-6648

## Beschreibung

Die vorliegende Erfindung betrifft substituierte C-Imidazo[1,2-a]pyridin-3-y1-methylamine sowie ihre physiologisch verträglichen Salze, Verfahren zu deren Herstellung, Arzneimittel enthaltend diese Verbindungen und die Verwendung von substituierten C-Imidazo[1,2-aJpyridin-3-yl-methylaminen zur Herstellung von Arzneimitteln.

Stickstoffmonoxid (NO) reguliert zahlreiche physiologische Prozesse, unter anderem die Neurotransmission, die Relaxation und Proliferation von glatter Muskulatur, die Adhäsion und Aggregation von Thrombozyten sowie die Gewebeverletzung und Entzündung. Aufgrund der Vielzahl von Signalfunktionen wird Stickstoffmonoxid mit einer Reihe von Krankheiten in Verbindung gebracht, beispielsweise in L. J. Ignarro, Angew. Chem. (1999), 111, Seiten 2002-2013 und in F. Murad, Angew. Chem. Int. Ed. (1999), 111, Seiten 1976-1989. Eine wichtige Rolle bei der therapeutischen Beeinflussung dieser Krankheiten spielt dabei das für die physiologische Bildung von Stickstoffmonoxid verantwortliche Enzym, die Stickstoffmonoxid-Synthase (NO-Synthase). Bislang wurden drei verschiedene lsoformen der NO-Synthase identifiziert, nämlich die beiden konstitutiven Formen nNO-Synthase und eNO-Synthase sowie die induzierbare Form iNO-Synthase (A. J. Hobbs, A. Higgs, S. Moncada, Annu. Rev. Pharmacol. Toxicol. (1999), 39, Seiten 191-220; I. C. Green, P.-E. Chabrier, DDT (1999), 4, Seiten 47-49; P.-E. Chabrier et al., Cell. Mol. Life Sci. (1999), 55, Seiten 1029-1035).

Die Hemmung der NO-Synthase eröffnet neue Therapieansätze für verschiedene Krankheiten, die mit Stickstoffmonoxid in Zusammenhang stehen (A. J. Hobbs et al., Annu. Rev. Pharmacol. Toxicol. (1999), 39, Seiten 191-220; I. C. Green, P.-E. Chabrier, DDT (1999),4, Seiten 47-49; P.-E. Chabrier et al., Cell. Mol. Life Sci. (1999), 55, Seiten 1029-1035), wie beispielsweise Migräne (L. L. Thomsen, J. Olesen, Clinical Neuroscience (1998), 5, Seiten 28-33; L. H. Lassen et al., The Lancet (1997), 349, 401-402), septischer Schock, neurodegenerative Erkrankungen wie Multiple Sklerose, Morbus Parkinson, Morbus Alzheimer oder Morbus Huntington, Entzündungen, Entzündungsschmerz, cerebrale Ischämie, Diabetes, Meningitis und Arteriosklerose. Darüber hinaus kann die Inhibierung der NO-Synthase einen Effekt auf die Wundheilung, auf Tumoren und auf die Angiogenese haben sowie eine unspezifische Immunität gegen Mikroorganismen bewirken (A. J. Hobbs et al., Annu. Rev. Pharmacol. Toxicol. (1999), 39, Seiten 191-220).

Bislang bekannte Wirkstoffe, die die NO-Synthase hemmen, sind neben L-NMMA und L-NAME- d.h. Analoga des L-Arginins, aus dem in-vivo unter Beteiligung von NO-Synthase Stickstoffmonoxid und Citrullin gebildet werden - u.a. S-Methyl-L-citrullin, Aminoguanidin, S-Methylisoharnstoff, 7-Nitroindazol und 2-Mercaptoethylguanidin (A. J. Hobbs et al., Annu. Rev. Pharmacol. Toxicol. (1999), 39, Seiten 191-220).

Eine Aufgabe der vorliegenden Erfindung bestand daher darin, neue Substanzen zur Verfügung zu stellen, die als Inhibitor auf die Stickstoffmonoxid-Synthase wirken. Insbesondere sollen sich die Arzneimittel zur Behandlung von Migräne, septischem Schock, neurodegenerativer Krankheiten, wie Multipler Sklerose, Morbus Parkinson, Morbus Alzheimer oder Morbus Huntington, Entzündungen, Entzündungsschmerz, cerebraler Ischämie, Diabetes, Meningitis, Arteriosklerose, Krebserkrankungen, Pilzerkrankungen oder zur Wundheilung eignen.

Es wurde gefunden, daß substituierte C-Imidazo[1,2-a]pyridin-3-yl-methylamine der nachstehenden allgemeinen Formel I als Inhibitoren der Stickstoffmonoxid-Synthase wirken und sich insbesondere zur Behandlung von Migräne, septischem Schock, neurodegenerativen Erkrankungen wie Multipler Sklerose, Morbus Parkinson, Morbus Alzheimer oder Morbus Huntington, Entzündungen, Entzündungsschmerz, cerebraler lschämie, Diabetes, Meningitis, Arteriosklerose, Krebserkrankungen, Pilzerkrankungen oder zur Wundheilung eignen.

Gegenstand der vorliegenden Erfindung sind daher substituierte C-Imidazo[1,2-a]pyridin-3-yl-methylamine der allgemeinen Formel 1, 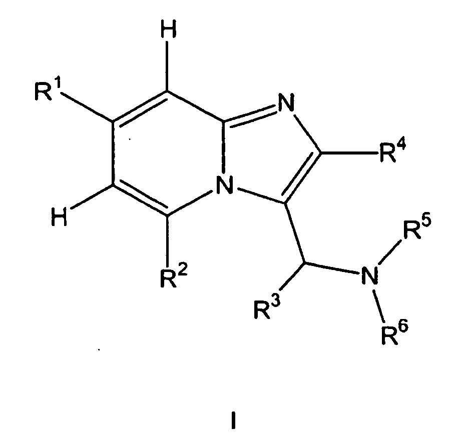 worin jeweils
- R¹: für OH, SH, NH₂, NHCH₃, N(CH₃)₂, CH₃, CH₂Cl, CH₂F, CHF₂, CF₃, OCH₃, OCH₂Cl, OCH₂F, OCHF₂, OCF₃, SCH₃, SCF₃, C₂H₅, CHClCH₃, CH₂CH₂Cl, CHFCH₃, CH₂CH₂F, CH₂CHF₂, CH₂CF₃, OC₂H₅, COOH, CH₂OH, CHOHCH₃, CH₂CH₂OH, CN, NO₂, F, Br, 1 oder Cl steht,
- R²: für H oder CH₃ steht,

- R³: für H; C₁₋₆-Alkyl, ein- oder mehrfach substituiert oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert oder unsubstituiert, verzweigt oder unverzweigt, oder jeweils gegebenenfalls ein- oder mehrfach substituiert oder unsubstituiert, verzweigt oder unverzweigt; C₃₋₈-Cycloalkyl, jeweils ein- oder mehrfach substituiert oder unsubstituiert, einfach ungesättigt oder gesättigt; Cyclopropyl, Cyclobutyl, Cyclopentyl, Cyclohexyl, Cycloheptyl, Cyclooctyl, 2-Methylcyclopropyl, Cyclopropylmethyl, 2-Methylcyclobutyl, 3-Methylcyclobutyl, Cyclobutylmethyl, jeweils substituiert oder unsubstituiert; oder Aryl oder Heteroaryl, jeweils substituiert oder unsubstituiert; oder COOR¹⁰ steht,
- R⁴: für H; C₁₋₁₂-Alkyl, ein- oder mehrfach substituiert oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, Heptyl, Octyl, Nonyl, Decyl, Undecyl, Dodecyl, jeweils ein- oder mehrfach substituiert oder unsubstituiert, verzweigt oder unverzweigt, oder jeweils gegebenenfalls ein- oder mehrfach substituiert oder unsubstituiert, verzweigt oder unverzweigt; C₃₋₈₋Cycloalkyl, jeweils ein- oder mehrfach substituiert oder
unsubstituiert, einfach ungesättigt oder gesättigt; Cyclopropyl, Cyclobutyl, Cyclopentyl, Cyclohexyl, Cycloheptyl, Cyclooctyl, 2-Methylcyclopropyl, Cyclopropylmethyl, 2-Methylcyclobutyl, 3-Methylcyclobutyl, Cyclobutylmethyl, jeweils substituiert oder unsubstituiert; oder einen Rest gemäß Formel II steht, 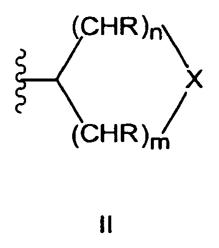 worin jeweils
- n: für eine Zahl wischen 0 und 6 steht,
- m: für eine Zahl wischen 0 und 6 steht,
1 ≤ m+n ≤ 6 ist,
- X: O, S, SO, SO₂ oder NR⁷ steht,
- R: unabhängig voneinander für H, F, Br, I, Cl, OH, SH, NH₂, NO₂, CH₃, C₂H₅, OCH₃, OC₂H₅, OCF₃ stehen,
- R⁵: für H; C₁₋₆-Alkyl, ein- oder mehrfach substituiert oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Di- methylpropyl, 1-Ethylpropyl oder Hexyl, jeweils ein- oder mehrfach substituiert oder unsubstituiert, verzweigt oder unverzweigt, oder jeweils gegebenenfalls ein- oder mehrfach substituiert oder unsubstituiert, verzweigt oder unverzweigt; oder C₃₋₈-Cycloalkyl, jeweils ein- oder mehrfach substituiert oder unsubstituiert, einfach ungesättigt oder gesättigt; Cyclopropyl, Cyclobutyl, Cyclopentyl, Cyclohexyl, Cycloheptyl, Cyclooctyl, 2-Methylcyclopropyl, Cyclopropylmethyl, 2-Methylcyclobutyl, 3-Methylcyclobutyl, Cyclobutylmethyl, jeweils substituiert oder unsubstituiert;
- R⁶: für H; C₁₋₆-Alkyl, ein- oder mehrfach substituiert oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert oder unsubstituiert, verzweigt oder unverzweigt, oder jeweils gegebenenfalls ein- oder mehrfach substituiert oder unsubstituiert, verzweigt oder unverzweigt; oder C₃₋₈-Cycloalkyl, jeweils ein- oder mehrfach substituiert oder unsubstituiert, einfach ungesättigt oder gesättigt; Cyclopropyl, Cyclobutyl, Cyclopentyl, Cyclohexyl, Cycloheptyl, Cyclooctyl, 2-Methylcyclopropyl, Cyclopropylmethyl, 2-Methylcyclobutyl, 3-Methylcyclobutyl, Cyclobutylmethyl, jeweils substituiert oder unsubstituiert; oder Aryl, Heteroaryl, C₁₋₄-Aryl oder C₁₋₄-Heteroaryl; substituiert oder ein- oder mehrfach substituiert; steht;
oder die Reste R⁵ und R⁶ zusammen einen Ring bilden und CH₂CH₂OCH₂CH₂, CH₂CH₂NR¹¹CH₂CH₂ oder (CH₂) ₃₋₆ bedeuten,
- R⁷: für H, einen C₁₋₆-Alkyl-Rest, vorzugsweise Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, 1-Ethylpropyl oder Hexyl, einen C₃-₈-Cycloalkyl-Rest, vorzugsweise Cyclopropyl, 2-Methylcyclopropyl, Cyclopropylmethyl, Cyclobutyl, 2-Methylcyclobutyl, 3-Methylcyclobutyl, Cyclobutylmethyl, Cyclopentyl, Cyclohexyl oder Cyclooctyl, einen Acylrest C(O)R⁸ oder einen Sulfonylrest S(O₂)R⁹ steht,
- R⁸: für H, einen C₁₋₆-Alkyl-Rest oder für einen Arylrest steht,
- R⁹: für H, einen C₁₋₆-Alkyl-Rest oder für einen Arylrest steht,
- R¹⁰: für H, einen C₁₋₆-Alkyl-Rest oder für einen Arylrest steht,
- R¹¹: für H, C₁₋₆-Alkyl oder C₃₋₈-Cycloalkyl, Aryl-, oder Heteroaryl, über C₁₋₃-Alkylen gebundenes Aryl oder Heteroaryl, Acyl C(O)R¹² oder Sulfonyl S(O₂)R¹³ steht,
- R¹²: für H, einen C₁₋₆-Alkyl-Rest oder für einen Arylrest steht,
- R¹³: für H, einen C₁₋₆-Alkyl-Rest oder für einen Arylrest steht,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
unter der Bedingung, dass die folgenden Verbindungen: 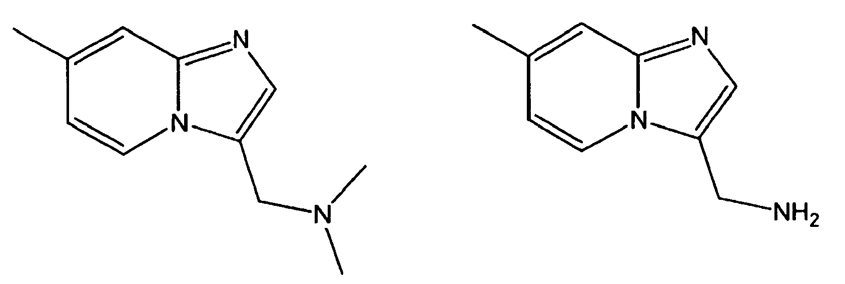 vom Schutz ausgenommen sind.

Der Ausdruck "C₁₋₁₂-Alkyl-Rest" umfaßt im Sinne der vorliegenden Erfindung acyclische gesättigte oder ungesättigte Kohlenwasserstoffreste, die verzweigt- oder geradkettig sowie unsubstituiert oder wenigstens einfach substituiert sein können, mit 1 bis 6 Kohlenstoff-Atomen. Das heißt, es werden neben C₁₋₁₂-Alkanylen auch C₂₋₁₂-Alkenyle und C₂₋₁₂-Alkinyle umfaßt, wobei die Alkenyle mindestens eine Kohlenstoff-Kohlenstoff-Doppelbindung, die Alkinyle mindestens eine Kohlenstoff-Kohlenstoff-Dreifachbindung aufweisen. Vorzugsweise ist der C₁₋₁₂-Alkyl-Rest ausgewählt aus der Gruppe Methyl, Ethyl, n-Propyl, 2-Propyl, n-Butyl, isoButyl, sec.-Butyl, tert.-Butyl, n-Pentyl, iso-Pentyl, neo-Pentyl, n-Hexyl, 2-Hexyl, n-Octyl, Ethenyl (Vinyl), Ethinyl, Propenyl (-CH₂CH=CH₂, -CH=CH-CH₃, -C(=CH₂)-CH₃), Propinyl (-CH-C=CH, -C≡C-CH₃), Butenyl, Butinyl, Pentenyl, Pentinyl, Hexenyl, Hexinyl, Octenyl und Octinyl.

Sofern der C₁₋₁₂-Alkyl-Rest einfach oder mehrfach substituiert vorliegt, ist (sind) ein oder mehrere Wasserstoffrest(e) bevorzugt durch einen Substituenten ausgewählt aus der Gruppe F, Cl, Br, 1, CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, 0-Heteroaryl, O-Alkyl-Aryl, 0-Alkyl-Heteroaryl, 0-Heterocyclyl, O-Alkyl-OH, CHO, C(=O)C₁-₆-Alkyl, C(=S)C₁₋₆-Alkyl, C(=O)Aryl, C(=S)Aryl,
C(=O)C₁₋₆-Alkyl-Aryl, 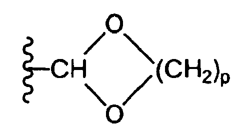 mit p = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂₋Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, Cycloalkyl, Aryl, Heteroaryl und Heterocyclyl ersetzt, wobei unter mehrfach substituierten C₁₋₆-Alkyl-Resten solche Reste zu verstehen sind, die entweder an verschiedenen Atomen oder an demselben Atom des C₁₋₆₋Alkyl-Restes mehrfach, z.B. zwei- oder dreifach, substituiert sind, beispielsweise dreifach am gleichen Kohlenstoff-Atom wie im Falle von CF₃ oder -CH₂CF₃ oder an verschiedenen Atomen wie im Falle von -CH(OH)-CH=CH-CHCl₂. Die Mehrfachsubstitution kann mit dem gleichen oder mit verschiedenen Substituenten erfolgen. Sofern der Substituent selbst eine Alkylgruppe aufweist, ist diese bevorzugt ausgewählt aus der Gruppe Methyl, Ethyl, CH₂-OH und CF₃.

Der Ausdruck "C₃₋₈-Cycloalkyl-Rest" umfaßt für die Zwecke der vorliegenden Erfindung cyclische Kohlenwasserstoffe mit 3 bis 8 Kohlenstoffatomen, die gesättigt oder ungesättigt, unsubstituiert oder wenigstens einfach substituiert sein können, wobei die Bindung des Cycloalkyl-Restes an das Grundgerüst der allgemeinen Formel I über jedes beliebige Ringglied des Cycloalkyl-Restes erfolgen kann. Bevorzugt ist der C₃₋₈-Cycloalkyl-Rest ausgewählt aus der Gruppe Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl. Besonders bevorzugt ist der C₃₋₈-Cycloalkyl-Rest ein Cyclohexyl-Rest.

Der Ausdruck "Aryl-Rest" bedeutet im Sinne der vorliegenden Erfindung aromatische Kohlenwasserstoffe, die auch mit weiteren gesättigten, zumindest teilweise ungesättigten oder aromatischen Ringsystemen kondensiert sein können, wobei die Bindung des Aryl-Restes an das Grundgerüst der allgemeinen Formel I über jedes beliebige Ringglied des Aryl-Restes erfolgen kann. Sofern der Aryl-Rest mehr als einen Substituenten aufweist, können diese gleich oder verschieden sein und in jeder beliebigen und möglichen Position des Aryl-Restes vorliegen. Vorzugsweise ist der Aryl-Rest ausgewählt aus der Gruppe von unsubstituierten oder wenigstens einfach substituiertem Phenyl, Anthracenyl, 1-Naphthyl und 2-Naphthyl. Besonders bevorzugt ist der Aryl-Rest ausgewählt aus der Gruppe Phenyl, 3-Hydroxyphenyl, 3-Methoxyphenyl, 2,3-Dihydroxyphenyl, 2,3-Dimethoxyphenyl und 1-Naphthyl.

Sofern der C₃₋₈-Cycloalkyl oder der Aryl-Rest einfach oder mehrfach substituiert ist, wir darunter bevorzugt die ein- oder mehrfache, z.B. zwei-, drei- oder vierfache, Substitution eines oder mehrerer Wasserstoffatome des Ringsystems mit einem Substituenten ausgewählt aus der Gruppe F, Cl, Br, I, CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Cycloalkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)-C₁₋₆₋Alkyl-Aryl, 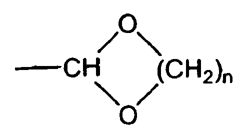 mit n = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂₋Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, S(O)-Alkyl, S(O)-Aryl, SO₂-Alkyl, SO₂₋Aryl, SO₂NH₂, SO₃H, CF₃, =O, =S; Alkyl, Cycloalkyl, Aryl, Heteroaryl und Heterocyclyl verstanden, wobei ein Substituent ggf. seinerseits substituiert sein kann. Die Mehrfachsubstitution erfolgt dabei mit gleichen oder unterschiedlichen Substituenten. Für "Aryl-Reste" sind besonders bevorzugte Substituenten ausgewählt aus der Gruppe F, CF₃, OH und O-CH₃. Für "Cycloalkyl-Reste" sind besonders bevorzugte Substituenten CO₂H oder CO₂Ethyl.

Der Ausdruck "Heteroaryl" steht im Sinne der vorliegenden Erfindung für einen 5-, 6- oder 7-gliedrigen cyclischen aromatischen Rest, der mindestens 1, ggf. auch 2 3, 4 oder 5 Heteroatome, aufweist, wobei die Heteroatome gleich oder verschieden sein können und wobei die Bindung an das Grundgerüst der allgemeinen Formel I über jedes beliebige und mögliche Ringglied des Heteroaryl-Restes erfolgen kann. Sofern der Heteroaryl-Rest mehr als einen Substituenten aufweist, können diese Heteroarylsubstituenten gleich oder verschieden sein und in jeder beliebigen und möglichen Position des Heteroaryls vorhanden sein. Der Heterocyclus kann auch mit weiteren gesättigten, zumindest teilweise ungesättigten oder aromatischen Ringsystemen kondensiert sein. Bevorzugte Heteroatome sind ausgewählt aus der Gruppe Stickstoff, Sauerstoff und Schwefel. Vorzugsweise ist der Heteroaryl-Rest ausgewählt aus der Gruppe von unsubstituiertem oder wenigstens einfach substituiertem Pyrrolyl, Furyl, Thienyl, Pyrazolyl, Imidazolyl, Pyridinyl, Pyridazinyl, Pyrimidinyl, Pyrazinyl, Pyranyl, Indolyl, Indazolyl, Purinyl, Pyrimidinyl, Indolizinyl, Chinolinyl, Isochinolinyl, Chinazolinyl, Carbazolyl, Phenazinyl und Phenothiazinyl. Besonders bevorzugte Heteroaryl-Reste sind ausgewählt aus der Gruppe Pyridin-2-yl, Pyridin-3-yl, Furan-2-yl, Furan-3-yl, 5-Hydroxymethylen-furan-2-yl, 5-Nitro-furan-2-yl, 5-[1,3]-dioxolan-furan-2-yl, 5-Carbonsäure-furan-2-yl, Thien-2-yl (2-Thiophen), Thien-3-yl (3-Thiophen) und 5-Carbonsäure-2-thiophen (5-Carbonsäurethien-2-yl).

Der Ausdruck "Heterocyclyl" umfaßt im Sinne der vorliegenden Erfindung einen 3-, 4-, 5-, 6- oder 7-gliedrigen cyclischen organischen. Rest, der mindestens 1, ggf. auch 2, 3, 4 oder 5 Heteroatome im Ringsystem aufweist, wobei die Heteroatome gleich oder verschieden sein können und der cyclische Rest gesättigt oder ungesättigt, nicht aber aromatisch ist und unsubstituiert oder wenigstens einfach substituiert sein kann. Die Bindung des Heterocyclyl-Restes an das Grundgerüst der allgemeinen Formel I kann über jedes beliebige Ringglied des Heterocyclyl-Restes erfolgen. Der Heterocyclyl-Rest kann auch Teil eines bi- oder polycyclischen Systems sein. Bevorzugte Heteroatome sind ausgewählt aus der Gruppe Stickstoff, Sauerstoff und Schwefel. Vorzugsweise ist der C₃₋₇-Heterocyclyl-Rest ausgewählt aus der Gruppe Tetrahydrofuryl, Tetrahydropyranyl, Pyrrolidinyl, Piperidinyl, Piperazinyl und Morpholinyl.

Sofern die erfindungsgemäßen Verbindungen der allgemeinen Formel I oder deren physiologisch verträgliche Salze wenigstens ein Asymmetriezentrum aufweisen, können sie in Form ihrer Racemate, ihrer reinen Enantiomeren, ihrer reinen Diastereomeren oder in Form eines Gemisches aus wenigstens zwei der vorstehend genannten Stereoisomeren vorliegen. Ebenso können die substituierten C-Imidazo[1,2-a]pyridin-3-yl-methylamine der allgemeinen Formel 1 auch in Form von Mischungen ihrer Enantiomeren oder Diastereomeren vorliegen. Diese Mischungen können die jeweiligen Stereoisomeren in jedem beliebigen Mischungsverhältnis aufweisen. Bevorzugt werden chirale substituierte C-Imidazo[1,2-a]pyridin-3-yl-methylamine der allgemeinen Formel I in enantiomerenreiner Form verwendet.

Im Sinne dieser Erfindung versteht man unter Alkyl- bzw. Cykloalkyl-Resten auch gesättigte und ungesättigte (aber nicht aromatische), verzweigte, unverzweigte und cyclische Kohlenwasserstoffe, die unsubstituiert oder ein- oder mehrfach substituiert sein können. Dabei steht C₁₋₂-Alkyl für C1- oder C2-Alkyl, C₁₋₃-Alkyl für C1-, C2- oder C3-Alkyl, C₁₋₄-Alkyl für C1-, C2-, C3- oder C4-Alkyl, C₁₋₅-Alkyl für C1-, C2-, C3-, C4- oder C5-Alkyl, C₁₋₆-Alkyl für C1-, C2-, C3-, C4-, C5- oder C6-Alkyl, C₁₋₇-Alkyl für C1-, C2-, C3-, C4-, C5-, C6- oder C7-Alkyl, C₁₋₈-Alkyl für C1-, C2-, C3-, C4-, C5-, C6-, C7- oder C8-Alkyl, C₁₋₁₀-Alkyl für C1-, C2-, C3-, C4-, C5-, C6-, C7-, C8,- C9- oder C10-Alkyl und C₁₋₁₈-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht C₃₋₄₋Cycloalkyl für C3- oder C4-Cycloalkyl, C₃_₅-Cycloalkyl für C3-, C4- oder C5-Cycloalkyl, C₃₋₆-Cycloalkyl für C3-, C4-, C5- oder C6-Cycloalkyl, C₃₋₇₋Cycloalkyl für C3-, C4-, C5-, C6- oder C7-Cycloalkyl, C₃₋₈-Cycloalkyl für C3-, C4-, C5-, C6-, C7- oder C8-Cycloalkyl, C₄₋₅-Cycloalkyl für C4- oder C5-Cycloalkyl, C₄-₆-Cycloalkyl für C4-, C5- oder C6-Cycloalkyl, C₄₋₇-Cycloalkyl für C4-, C5-, C6- oder C7-Cycloalkyl, C₅₋₆-Cycloalkyl für C5- oder C6-Cycloalkyl und C₅₋₇-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, CHF₂, CF₃ oder CH₂OH sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung auch die Substitution mindestens eines (gegebenenfalls auch mehrerer) Wasserstoffreste(s) durch F, Cl, Br, I, NH₂, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCl₂. Besonders bevorzugte Substituenten sind hier F, Cl und OH. In Bezug auf Cycloalkyl kann der Wasserstoffrest auch durch OC₁₋₃-Alkyl oder C₁₋₃-Alkyl (jeweils ein- oder mehrfach substituiert oder unsubstituiert), insbesondere Methyl, Ethyl, n-Propyl, i-Propyl, CF₃, Methoxy oder Ethoxy, ersetzt sein.

Unter dem Begriff (CH₂)₃₋₆ ist -CH₂-CH₂-CH₂-, -CH₂-CH₂-CH₂-CH₂-, -CH₂₋CH₂-CH₂-CH₂-CH₂- und CH₂CH₂-CH₂-CH₂-CH₂-CH₂- zu verstehen, unter (CH₂)₁₋₄ ist -CH₂-, -CH₂-CH₂-, -CH₂-CH₂-CH₂- und -CH₂-CH₂-CH₂-CH₂- zu verstehen, unter (CH₂)₄₋₅ ist -CH₂-CH₂-CH₂-CH₂- und -CH₂-CH₂-CH₂-CH₂₋CH₂- zu verstehen, etc.

Unter einem Aryl-Rest werden auch Ringsysteme mit mindestens einem armomatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden auch heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo-1,2,5 thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert auch die Substitution des Aryls oder Heteroaryls mit R²³, OR²³ einem Halogen, vorzugsweise F und/oder Cl, einem CF₃, einem CN, einem NO₂, einem NR²⁴R²⁵, einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen.

Dabei steht der Rest R²³ für H, einen C₁₋₁₀-Alkyl-, vorzugsweise einen C₁₋₆-Alkyl-, einen Aryl- oder Heteroaryl- oder für einen über eine C₁₋₃₋Alkylen-Gruppe gebundenen Aryl- oder Heteroaryl-Rest, wobei diese Aryl und Heteroarylreste nicht selbst mit Aryl- oder Heteroaryl-Resten substituiert sein dürfen,
die Reste R²⁴ und R²⁵, gleich oder verschieden, für H, einen C₁₋₁₀-Alkyl-, vorzugsweise einen C₁₋₆-Alkyl-, einen Aryl-, einen Heteroaryl- oder einen über eine C₁₋₃-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste R²⁴ und R²⁵ bedeuten zusammen CH₂CH₂OCH₂CH₂, CH₂CH₂NR²⁶CH₂CH₂ oder (CH₂)₃₋₆, und
der Rest R²⁶ für H, einen C₁₋₁₀-Alkyl-, vorzugsweise einen C₁₋₆-Alkyl-, einen Aryl-, oder Heteroaryl- Rest oder für einen über eine C₁₋₃-AlkylenGruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist im Sinne dieser Erfindung jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und lonen zu verstehen, insbesondere Komplexe, die über ionische Wechselwirkungen komplexiert sind.

Unter dem Begriff des physiologisch verträglichen Salzes (insbesondere mit Kationen oder Basen) versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit NH₄⁺, insbesondere aber (Mono-) oder (Di-) Natrium-, (Mono-) oder (Di-) Kalium-, Magnesium- oder Calzium-Salze.

Unter dem Begriff des physiologisch verträglichen Salzes (insbesondere mit Anionen oder Säuren) versteht man im Sinne dieser Erfindung weiter Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydrolb6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz.

Geeignete Salze im Sinne dieser Erfindung und in jeder beschriebenen Verwendung und jedem der beschriebenen Arzneimittel sind Salze des jeweiligen Wirkstoffes mit anorganischen bzw. organischen Säuren und/oder einem Zuckeraustauschstoff wie Saccharin, Cyclamat oder Acesulfam. Besonders bevorzugt ist jedoch das Hydrochlorid.

In einer bevorzugten Ausführungsform des
C-Imidazo[1,2-a]pyridin-3-yl-methylamins steht R⁴
für C₁₋₁₂-Alkyl, ein- oder mehrfach substituiert oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, Heptyl, Octyl, Nonyl, Decyl, Undecyl, Dodecyl, jeweils ein- oder mehrfach substituiert oder unsubstituiert, verzweigt oder unverzweigt, oder jeweils gegebenenfalls ein- oder mehrfach substituiert oder unsubstituiert, verzweigt oder unverzweigt; C₃₋₈₋Cycloalkyl, jeweils ein- oder mehrfach substituiert oder unsubstituiert, einfach ungesättigt oder gesättigt; Cyclopropyl, Cyclobutyl, Cyclopentyl, Cyclohexyl, Cycloheptyl, Cyclooctyl, 2-Methylcyclopropyl, Cyclopropylmethyl, 2-Methylcyclobutyl, 3-Methylcyclobutyl, Cyclobutylmethyl, jeweils substituiert oder unsubstituiert; oder einen Rest gemäß Formel II, 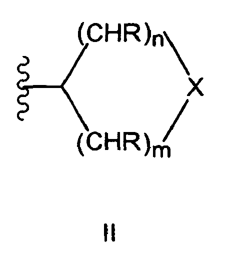 worin jeweils
- n: für eine Zahl wischen 0 und 6 steht,
- m: für eine Zahl wischen 0 und 6 steht,
1 ≤ m+n ≤ 6 ist,
- X: O, S, SO, SO₂ oder NR⁷ steht,
- R: unabhängig voneinander für H, F, Br, I, Cl, OH, SH, NH₂, NO₂, CH₃, C₂H₅, OCH₃, OC₂H₅, OCF₃ stehen.

In einer bevorzugten Ausführungsform des C-Imidazo[1,2-a]pyridin-3-yl-methylamins steht R³
für C₁₋₆-Alkyl, ein- oder mehrfach substituiert oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethytpropyl oder Hexyl, jeweils ein- oder mehrfach substituiert oder unsubstituiert, verzweigt oder unverzweigt, oder jeweils gegebenenfalls ein- oder mehrfach substituiert oder unsubstituiert, verzweigt oder unverzweigt; C₃₋₈-Cycloalkyl, jeweils ein- oder mehrfach substituiert oder unsubstituiert, einfach ungesättigt oder gesättigt; Cyclopropyl, Cyclobutyl, Cyclopentyl, Cyclohexyl, Cycloheptyl, Cyclooctyl, 2-Methylcyclopropyl, Cyclopropylmethyl, 2-Methylcyclobutyl, 3-Methylcyclobutyl, Cyclobutylmethyl, jeweils substituiert oder unsubstituiert; oder Aryl oder Heteroaryl, jeweils substituiert oder unsubstituiert; oder COOR¹⁰.

In einer bevorzugten Ausführungsform des *C*-Imidazo[1,2-a]pyridin-3-yl-methylamins steht R¹ für C₂H₅, CH₃, CF₃ oder Cl, insbesondere für CH₃, CF₃ oder Cl, vorzugsweise für CH₃.

In einer bevorzugten Ausführungsform des C-Imidazo[1,2-a]pyridin-3-yl-methylamins steht R⁵ für H; C₁₋₆-Alkyl, ein- oder mehrfach substituiert oder unsubstituiert, gesättigt oder ungesättigt, verzweigt oder unverzweigt; oder Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert oder unsubstituiert, verzweigt oder unverzweigt, oder jeweils gegebenenfalls ein- oder mehrfach substituiert oder unsubstituiert, verzweigt oder unverzweigt.

In einer bevorzugten Ausführungsform des C-Imidazo[1,2-a]pyridin-3-yl-methylamins steht R³ für H; C₁₋₆-Alkyl, ein- oder mehrfach substituiert oder unsubstituiert, gesättigt oder ungesättigt, verzweigt oder unverzweigt; oder Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert oder unsubstituiert, verzweigt oder unverzweigt, oder jeweils gegebenenfalls ein- oder mehrfach substituiert oder unsubstituiert, verzweigt oder unverzweigt;
vorzugsweise
für C₁₋₆-Alkyl, ein- oder mehrfach substituiert oder unsubstituiert, gesättigt oder ungesättigt, verzweigt oder unverzweigt; oder Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert oder unsubstituiert, verzweigt oder unverzweigt, oder jeweils gegebenenfalls ein- oder mehrfach substituiert oder unsubstituiert, verzweigt oder unverzweigt.

In einer bevorzugten Ausführungsform des C-Imidazo[1,2-a]pyridin-3-yl-methylamins steht R² für H.

In einer bevorzugten Ausführungsform des *C*-Imidazo[1,2-a]pyridin-3-yl-methylamins steht R⁶ für H; C₁₋₆-Alkyl, ein- oder mehrfach substituiert oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert oder unsubstituiert, verzweigt oder unverzweigt, oder jeweils gegebenenfalls ein- oder mehrfach substituiert oder unsubstituiert, verzweigt oder unverzweigt.

In einer bevorzugten Ausführungsform des C-Imidazo[1,2-aJpyridin-3-yl-methylamins bilden die Reste R⁵ und R⁶ zusammen einen Ring und bedeuten CH₂CH₂OCH₂CH₂, CH₂CH₂NR¹¹CH₂CH₂ oder (CH₂) ₃₋₆.

Die Herstellung der substituierten C-Imidazo[1,2-a]pyridin-3-yl-methylamine der allgemeinen Formel I kann nach üblichen, dem Fachmann bekannten Methoden erfolgen.

Vorzugsweise erfolgt die Herstellung der erfindungsgemäßen Verbindungen der allgemeinen Formel 1 schrittweise, im ersten Schritt durch Umsetzung eines substituierten 2-Aminopyridins der allgemeinen Formel III, worin R¹ und R² die Bedeutung gemäß der oben angegebenen allgemeinen Formel I haben, 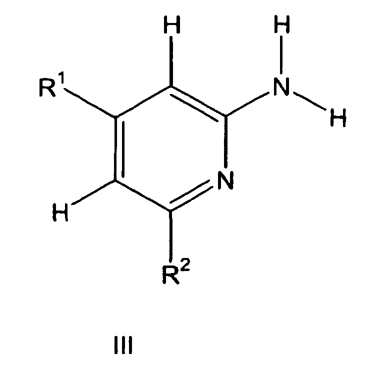 vorzugsweise in Lösung mit einer α-Halogencarbonylverbindung der allgemeinen Formel IV, 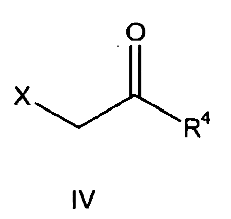 worin die Reste R³ und R⁴ die Bedeutung gemäß der allgemeinen Formel I haben und X für Halogen, vorzugsweise für Cl, Br oder 1 steht, unter Abspaltung von Wasser und Halogenwasserstoff und Bildung des lntermediats der Formel V in 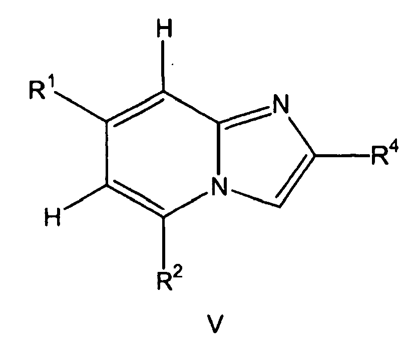 und anschließende Aminomethylierung dieses Intermediats V in einem zweiten Schritt.

Vorteilhafterweise wird der erste Verfahrensschritt unter Bedingungen durchgeführt, bei denen Wasser und/oder Halogenwasserstoff vorzugsweise kontinuierlich aus dem Reaktionsgemisch entfernt werden.

Halogenwasserstoff kann bevorzugt durch Zugabe löslicher oder unlöslicher organischer oder anorganischer Basen gebunden und so aus dem Reaktionsgemisch entfernt werden.
Wasser kann bevorzugt durch azeotrope Destillation oder durch Zusatz von Trockenmitteln oder hygroskopischen Substanzen aus dem Reaktionsgemisch entzogen werden.

Die Herstellung der erfindungsgemäßen Intermediate der allgemeinen Formel V nach dem obenstehenden Verfahren, mit oder ohne Lösungsmittel, bei Temperaturen von mehr als 100 °C stellt eine weitere Möglichkeit dar, Wasser aus dem Reaktionsgemisch zu entfernen.

Besonders bevorzugt ist die Herstellung der erfindungsgemäßen Intermediate der allgemeinen Formel V durch Umsetzung substituierter 2-Aminopyridine der allgemeinen Formel 111 mit α-Halogencarbonylverbindungen der allgemeinen Formel IV, worin X für Br steht, in siedendem, wasserfreien Ethanol.

Ebenfalls bevorzugt ist die Herstellung der erfindungsgemäßen Intermediate der allgemeinen Formel V durch Umsetzung substituierter 2-Aminopyridine der allgemeinen Formel III mit α-Halogencarbonylverbindungen der allgemeinen Formel IV, worin X für Br oder Cl steht, in siedendem, wasserfreien Di- und/oder Trichlormethan am Wasserabscheider.

Die substituierten 2-Aminopyridine der allgemeinen Formel III sowie die α-Halogencarbonylverbindungen der allgemeinen Formel IV sind allgemein am Markt erhältlich oder können nach üblichen, dem Fachmann bekannten Methoden hergestellt werden.

Der zweite Verfahrensschritt ist die Aminomethylierung der erfindungsgemäßen Intermediate der allgemeinen Formel V durch Umsetzung mit lminiumsalzen der Formel VI, die sowohl zuvor separat als auch in situ hergestellt werden können.

Die Imminiumsalz der allgemeinen Formel VI, 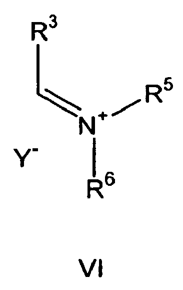 worin Y vorzugsweise Cl⁻, AlCl₄⁻, Br⁻ oder 1- bedeutet, können nach literaturbekannten Verfahren durch Umsetzung von Aminalen der allgemeinen Formel VII 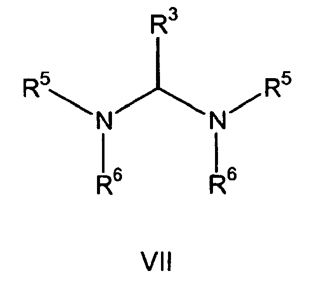 mit Säurechloriden, beispielsweise Acetylchlorid oder Thionylchlorid, hergestellt werden (Houben-Weyl - Methoden der Organischen Chemie, E21 b, 1995, S. 1925-1929). Für R³ = COOR₁₀ hingegen, ist das entsprechende Verfahren bei Merla et al. beschrieben (Merla B., Grumbach H.-J., Risch N.; Synthesis 1998, 1609 -1614). Beide Artikel sind vollinhaltlich Teil der Beschreibung.

Die Imminiumsalze der allgemeinen Formel VI müssen dabei nicht isoliert werden, sondern können in situ mit efindungsgemäßen Intermediaten der allgemeinen Formel V zu erfindungsgemäßen Substanzen der allgemeinen Formel I umgesetzt werden.

Zur Einführung eines Dimethylaminomethylrest ist außerdem die Umsetzung erfindungsgemäßer Intermediate der allgemeinen Formel V mit Paraformaldehyd und Dimethylammoniumchlorid bei Temperaturen zwischen 50 und 150 °C ein geeignetes Verfahren.

Die erfindungsgemäßenen substituierten *C*-Imidazo[1,2-a]pyridin-3-yl-methylamine der allgemeinen Formel 1 können nach dem zu ihrer Herstellung eingesetzten Verfahren sowohl als freie Base wie auch als Salz isoliert werden. Die freie Base der jeweiligen Verbindung der allgemeinen Formel 1 wird üblicherweise nach erfolgter Umsetzung gemäß dem oben beschriebenen efindungsgemäßen Verfahren und ggf. anschließender Aufarbeitung nach üblichen, dem Fachmann bekannten Methoden erhalten. Die so erhaltene oder in-situ ohne Isolierung gebildete freie Base der jeweiligen Verbindung der allgemeinen Formel I kann dann, beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in das entsprechende, physiologisch verträgliche Salz übergeführt werden.

Die Überführung der jeweiligen Verbindung der allgemeinen Formel 1 kann bevorzugt auch durch Versetzen der in einem geeigneten organischen Lösungsmittel, wie z.B. Butan-2-on (Methylethylketon), gelösten Verbindung der allgemeinen Formel I als freie Base mit Trimethylsilylchlorid (TMSCI) erhalten werden.

Sofern die erfindungsgemäßen substituierte *C*-Imidazo[1,2-a]pyridin-3-yl-methylamine der allgemeinen Formel 1 nach dem erfindungsgemäßen Herstellungsverfahren in Form ihrer Racemate oder anderer Mischungen ihrer verschiedenen Enantiomeren und/oder Diastereomeren erhalten wird, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die Verbindungen sind bzw. erscheinen toxikologisch unbedenklich.

Ein weiterer Gegenstand der vorliegenden Erfindung ist daher ein Arzneimittel enthaltend mindestens ein erfindungsgemäßes substituiertes C-Imidazo[1,2-a]pyridin-3-yl-methylamin bzw. ein substituiertes C-Imidazo[1,2-a]pyridin-3-yl-methylamin gemäß der oben angebenen allgemeinen Formel 1, sowie gegebenenfalls Hilfsstoffe bzw. weitere Hilfsstoffe.

Ein weiterer Gegenstand der vorliegenden Erfindung ist die Verwendung wenigstens eines erfindungsgemäßen substituierten C-Imidazo[1,2-a]pyridin-3-yl-methylamins bzw. eines substituierten C-Imidazo[1,2-a]pyridin-3-yl-methylamins gemäß der oben angebenen allgemeinen Formel 1 - gegebenenfalls als Inhibitor der Stickstoffmonoxid-Synthase - zur Herstellung eines Arzneimittels zur Behandlung von Migräne, septischem Schock, neurodegenerativen Erkrankungen, wie Multipler Sklerose, Morbus Parkinson, Morbus Alzheimer oder Morbus Huntington, Entzündungsschmerz, cerebraler Ischämie, Diabetes, Meningitis, Arteriosklerose, Krebserkrankungen oder zur Wundheilung.

Die entsprechenden Arzneimittel können als flüssige, halbfeste oder feste Arzneiformen, beispielsweise in Form von Injektionslösungen, Tropfen, Säften, Sirupen, Sprays, Suspensionen, Granulaten, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, vorliegen und als solche auch verabreicht werden.

Neben wenigstens einem erfindungsgemäßen substituierten C-Imidazo[1,2-a]pyridin-3-yl-methylamin der allgemeinen Formel I enthalten die erfindungsgemäßen Arzneimittel üblicherweise weitere physiologisch verträgliche pharmazeutische Hilfsstoffe, die bevorzugt ausgewählt sind aus der Gruppe von Trägermaterialien, Füllstoffen, Lösungsmittel, Verdünnungsmittel, oberflächenaktiven Stoffen, Farbstoffen, Konservierungsstoffen, Sprengmittel, Gleitmittel, Schmiermittel, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäßen Verbindungen der allgemeinen Formel I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Formel 1 auch verzögert freisetzen.

Die Herstellung der Arzneimittel erfolgt mit Hilfe von üblichen, dem Fachmann bekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt somit als Teil der Offenbarung.

Die an den Patienten zu verabreichende Menge der jeweiligen Verbindung der allgemeinen Formel I kann variieren und ist beispielsweise abhängig vom Gewicht oder dem Alter des Patienten sowie von der Applikationsart, der Indikation und dem Schweregrad der Erkrankung. Üblicherweise werden 0,1 bis 5000 mg/kg, vorzugsweise 1 bis 500 mg/kg, besonders bevorzugt 2 bis 250 mg pro kg Körpergewicht des Patienten wenigstens einer Verbindung der allgemeinen Formel I appliziert.

### Beispiele:

### Beispiel 1:

### Synthese von 2,7-Dimethyl-imidazo[1,2-a]pyridin-3-ylmethyl)-dimethyl-amin

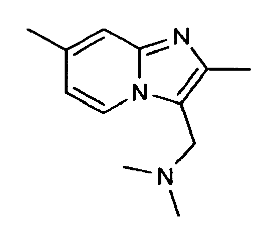

Zu 0,83 g Formaldehyd (Formalin 37 %-tig) und 1,30 ml Dimethylamin (40 %-tig in Wasser) in 1,41 ml Eisessig gab man bei 0 °C unter Stickstoffatmosphäre 1,50 g 2,7-Dimethyl-imidazo[1,2-α]pyridin, erhitzte das Reaktionsgemisch zwei Stunden bei 50 °C und rührte über Nacht bei einer Temperatur von 20 bis 25 °C. Zur weiteren Reinigung wurde das Reaktionsgemisch mit 10 %-iger Natronlauge alkalisch gestellt und mit Diethylether extrahiert. Die organischen Phasen wurden vereinigt und über Natriumsulfat getrocknet. Nach Entfernung des organischen Lösungsmittels durch Destillation wurden 1,70 g des Rohproduktes erhalten. Nach Reinigung durch Säulenchromatographie erhielt man 177 mg des Produktes als farbloses Öl. 177 mg der Base wurden mit 1 ml Ethylmethylketon verdünnt und durch Zugabe von 0,009 ml Wasser und 0,121 ml Chlortrimethylsilan und anschließendem Rühren über Nacht als Hydrochlorid gefällt. Man erhielt 170 mg des 2,7-Dimethyl-imidazo[1,2-α]pyridin-3-ylmethyl)-dimethyl-amin-hydrochlorids (entsprechen 6,5 % der theoretischen Menge) als farblosen Feststoff.

### Beispiel 2: Molekularpharmakologische Untersuchung

Der lC50-Wert der Beispielverbindung wurde in einem Citrullin-Assay bestimmt. Dieser Assay wurde wie von D. S. Bredt und S. H. Snyder (Proc. Natl. Acad. Sci. USA (1990), 87, 682-685) beschrieben durchgeführt. Die Ergebnisse von Beispielverbindung im Citrullin-Assay ist in Tabelle 1 wiedergegeben.

**Tabelle 1:**

| Verbindung Nr. | Hemmung der Stickstoffmonoxid-Synthase IC50 [µm] |
|---|---|
| 1 | 4,0 |

## Patentansprüche

1. Substituiertes C-Imidazo[1,2-a]pyridin-3-yl-methylamin der allgemeinen Formel I, 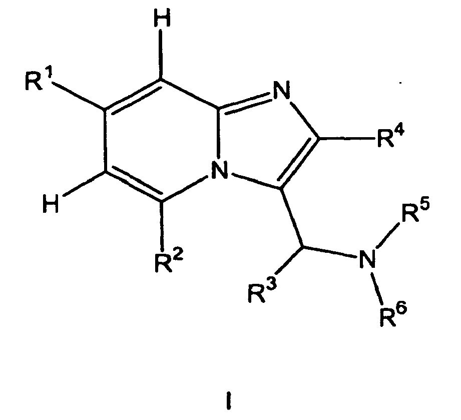 worin jeweils
R¹ für OH, SH, NH₂, NHCH₃, N(CH₃)₂, CH₃, CH₂Cl, CH₂F, CHF₂, CF₃, OCH₃, OCH₂Cl, OCH₂F, OCHF₂, OCF₃, SCH₃, SCF₃, C₂H₅, CHClCH₃, CH₂CH₂Cl, CHFCH₃. CH₂CH₂F, CH₂CHF₂, CH₂CF₃, OC₂H₅, COOH, CH₂OH, CHOHCH₃, CH₂CH₂OH, CN, NO₂, F, Br, I oder Cl steht,
R² für H oder CH₃ steht,
R³ für H; C₁₋₆-Alkyl, ein- oder mehrfach substituiert mit F, Cl, Br, I, NH₂, SH oder OH, oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert mit F, Cl, Br, I, NH₂, SH oder OH, oder unsubstituiert, verzweigt oder unverzweigt; C₃₋₈-Cycloalkyl, jeweils ein- oder mehrfach substituiert mit F, Cl, Br, I, NH₂, SH oder OH, oder unsubstituiert, einfach ungesättigt oder gesättigt; Cyclopropyl, Cyclobutyl, Cyclopentyl, Cyclohexyl, Cycloheptyl, Cyclooctyl, 2-Methylcyclopropyl, Cyclopropylmethyl, 2-Methylcyclobutyl, 3-Methylcyclobutyl, Cyclobutylmethyl, jeweils substituiert mit CO₂H oder CO₂Ethyl, oder unsubstituiert; oder Phenyl, Naphthyl oder Anthracenyl, jeweils substituiert mit F, CF₃, OH oder OCH₃ oder unsubstituiert; oder Pyridin-2-yl, Pyridin-3-yl, Furan-2-yl, Furan-3-yl, 5-Hydroxymethylen-furan-2-yl, 5-Nitrofuran-2-yl, 5-[1,3]-dioxolan-furan-2-yl, 5-Carbonsäure-furan-2-yl, Thien-2-yl, Thien-3-yl und 5-Carbonsäure-2-thiophen; oder COOR¹⁰ steht,
R⁴ für H; C₁₋₁₂-Alkyl, ein- oder mehrfach substituiert mit F, Cl, Br, I, NH₂, SH oder OH, oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, Heptyl, Octyl, Nonyl, Decyl, Undecyl, Dodecyl, jeweils ein- oder mehrfach substituiert mit F, Cl, Br, I, NH₂, SH oder OH, oder unsubstituiert, verzweigt oder unverzweigt; C₃₋₈₋Cycloalkyl, jeweils ein- oder mehrfach substituiert mit F, CI, Br, I, NH₂, SH oder OH oder unsubstituiert, einfach ungesättigt oder gesättigt; Cyclopropyl, Cyclobutyl, Cyclopentyl, Cyclohexyl, Cycloheptyl, Cyclooctyl, 2-Methyl- cyclopropyl, Cyclopropylmethyl, 2-Methylcyclobutyl, 3-Methylcyclobutyl, Cyclobutylmethyl, jeweils substituiert mit CO₂H oder CO₂Ethyl, oder unsubstituiert; oder einen Rest gemäß Formel II steht, 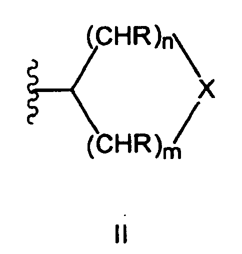
worin jeweils
n für eine Zahl zwischen 0 und 6 steht,
m für eine Zahl zwischen 0 und 6 steht,
1 ≤ m+n ≤ 6 ist,
X O, S, SO, SO₂ oder NR⁷ steht,
R unabhängig voneinander für H, F, Br, 1, Cl, OH, SH, NH₂, NO₂, CH₃, C₂H₅, OCH₃, OC₂H₅, OCF₃ stehen,
R⁵ für H; C₁₋₆-Alkyl, ein- oder mehrfach substituiert mit F, Cl, Br, I, NH₂, SH oder OH, oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert mit F, Cl, Br, I, NH₂. SH oder OH, oder unsubstituiert, verzweigt oder unverzweigt; oder C₃₋₈-Cycloalkyl, jeweils ein- oder mehrfach substituiert mit F, Cl, Br, 1, NH₂, SH oder OH, oder unsubstituiert, einfach ungesättigt oder gesättigt; Cyclopropyl, Cyclobutyl, Cyclopentyl, Cyclohexyl, Cycloheptyl, Cyclooctyl, 2-Methylcyclopropyl, Cyclopropylmethyl, 2-Methylcyclobutyl, 3-Methylcyclobutyl, Cyclobutylmethyl, jeweils substituiert mit CO₂H oder CO₂Ethyl, oder unsubstituiert;
R⁶ für H; C₁₋₆-Alkyl, ein- oder mehrfach substituiert mit F, Cl, Br, 1, NH₂, SH oder OH, oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert mit F, Cl, Br, 1, NH₂, SH oder OH, oder unsubstituiert, verzweigt oder unverzweigt; oder C₃₋₈-Cycloalkyl, jeweils ein- oder mehrfach substituiert mit F, Cl, Br, 1, NH₂, SH oder OH, oder unsubstituiert, einfach ungesättigt oder gesättigt; Cyclopropyl, Cyclobutyl, Cyclopentyl, Cyclohexyl, Cycloheptyl, Cyclooctyl, 2-Methylcyclopropyl, Cyclopropylmethyl, 2-Methytcyclobutyl, 3-Methylcyclobutyl, Cyclobutyimethyt, jeweils substituiert mit CO₂H oder CO₂Ethyl, oder unsubstituiert; oder Phenyl, Naphthyl oder Anthracenyl, jeweils substituiert mit F, CF₃, OH oder OCH₃ oder unsubstituiert; oder Pyridin-2-yl, Pyridin-3-yl, Furan-2-yl, Furan-3-yl, 5-Hydroxymethylen-furan-2-yl, 5-Nitrofuran-2-yl, 5-[1,3]-dioxolan-furan-2-yl, 5-Carbonsäure-furan-2- yl, Thien-2-yl, Thien-3-yl und 5-Carbonsäure-2-thiophen; steht;
oder die Reste R⁵ und R⁶ zusammen einen Ring bilden und CH₂CH₂OCH₂CH₂, CH₂CH₂NR¹¹CH₂CH₂ oder (CH₂) ₃₋₆ bedeuten,
R⁷ für H, einen C₁₋₆-Alkyl-Rest, vorzugsweise Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, 1-Ethylpropyl oder Hexyl, einen C₃₋₈-Cycloalkyl-Rest, vorzugsweise Cyclopropyl, 2-Methylcyclopropyl, Cyclopropylmethyl, Cyclobutyl, 2-Methylcyclobutyl, 3-Methylcyclobutyl, Cyclobutylmethyl, Cyclopentyl, Cyclohexyl oder Cyclooctyl, einen Acylrest C(O)R⁸ oder einen Sulfonylrest S(O₂)R⁹ steht,
R⁸ für H, einen C₁₋₆-Alkyl-Rest oder für einen Phenyl-, Naphthyl- oder Anthracenylrest steht,
R⁹ für H, einen C₁₋₆-Alkyl-Rest oder für einen Phenyl-, Naphthyl- oder Anthracenylrest steht,
R¹⁰ für H, einen C₁₋₆-Alkyl-Rest oder für einen Phenyl-, Naphthyl- oder Anthracenylrest steht,
R¹¹ für H, C₁₋₆-Alkyl oder C₃₋₈-Cycloalkyl oder einen Phenyl-, Naphthyl- oder Anthracenylrest; oder Pyridin-2-yl, Pyridin-3-yl, Furan-2-yl, Furan-3-yl, 5-Hydroxymethylen-furan-2-yl, 5-Nitrofuran-2-yl, 5-[1,3J-dioxolan-furan-2-yl, 5-Carbonsäure-furan-2-yl, Thien-2-yl, Thien-3-yl und 5-Carbonsäure-2-thiophen; über C₁₋₃-Alkylen gebundenes Phenyl, Naphthyl oder Anthracenyl, oder Pyridin-2-yl, Pyridin-3-yl, Furan-2-yl, Furan-3-yl, 5-Hydroxymethylen-furan-2-yl, 5-Nitro-furan-2-yl, 5-[1,3]-dioxolan-furan-2-yl, 5-Carbonsäure-furan-2-yl, Thien-2-yl, Thien-3-yl und 5-Carbonsäure-2-thiophen; Acyl C(O)R¹² oder Sulfonyl S(O₂)R ¹³ steht,
R¹² für H, einen C₁₋₆-Alkyl-Rest oder für einen Phenyl-, Naphthyl- oder Anthracenylrest steht,
R¹³ für H, einen. C₁₋₆-Alkyl-Rest oder für einen Phenyl-, Naphthyl- oder Anthracenylrest steht,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
unter der Bedingung, dass die folgenden Verbindungen: 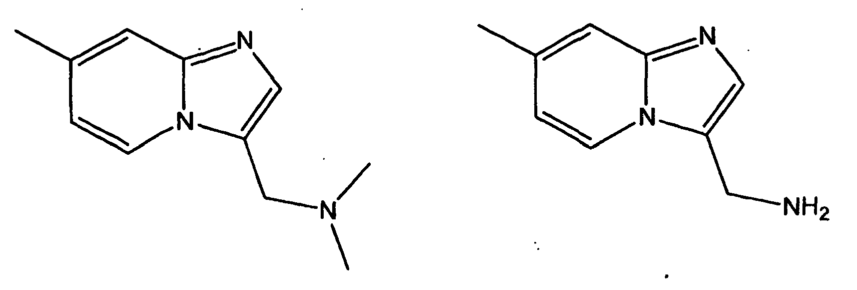 vom Schutz ausgenommen sind.

2. C-Imidazo[1,2-a]pyridin-3-yl-methyiamin gemäß Anspruch 1, **dadurch gekennzeichnet, daß** R⁴
für C₁₋₁₂-Alkyl, ein- oder mehrfach substituiert mit F, Cl, Br, I, NH₂, SH oder OH, oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, Heptyl, Octyl, Nonyl, Decyl, Undecyl, Dodecyl, jeweils ein- oder mehrfach substituiert mit F, Cl, Br, 1, NH₂, SH oder OH, oder unsubstituiert, verzweigt oder unverzweigt; C₃₋₈₋Cycloalkyl, jeweils ein- oder mehrfach substituiert mit F, Cl, Br, I, NH₂, SH oder OH, oder unsubstituiert, einfach ungesättigt oder gesättigt; Cyclopropyl, Cyclobutyl, Cyclopentyl, Cyclohexyl, Cycloheptyl, Cyclooctyl, 2-Methylcyclopropyl, Cyclopropylmethyl, 2-Methylcyclobutyl, 3-Methylcyclobutyl, Cyclobutylmethyl; oder einen Rest gemäß Formel I steht, 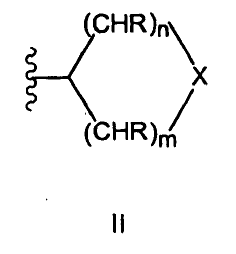 worin jeweils
n für eine Zahl zwischen 0 und 6 steht,
m für eine Zahl zwischen 0 und 6 steht,
1 ≤ m+n ≤ 6 ist,
X O, S, SO, SO₂ oder NR⁷ steht,
R unabhängig voneinander für H, F, Br, 1, Cl, OH, SH, NH₂, NO₂, CH₃, C₂H₅, OCH₃, OC₂H₅, OCF₃ stehen.

3. C-Imidazo[1,2-a]pyridin-3-yl-methylamin gemäß einem der Ansprüche 1 oder 2, **dadurch gekennzeichnet, daß** R³
für C₁₋₆-Alkyl, ein- oder mehrfach substituiert mit F, Cl, Br, I, NH₂, SH oder OH; oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert mit F, Cl, Br, I, NH₂, SH oder OH, oder unsubstituiert, verzweigt oder unverzweigt; C₃₋₈-Cycloalkyl, jeweils ein- oder mehrfach substituiert mit F, Cl, Br, I, NH₂, SH oder OH, oder unsubstituiert, einfach ungesättigt oder gesättigt; Cyclopropyl, Cyclobutyl, Cyclopentyl, Cyclohexyl, Cycloheptyl, Cyclooctyl, 2-Methylcyclopropyl, Cyclopropylmethyl, 2-Methylcyclobutyl, 3-Methylcyclobutyl, Cyclobutylmethyl; oder Phenyl, Naphthyl oder Anthracenyl, jeweils substituiert mit F, CF₃, OH oder OCH₃ oder unsubstituiert; oder Pyridin-2-yl, Pyridin-3-yl, Furan-2-yl, Furan-3-yl, 5-Hydroxymethylen-furan-2-yl, 5-Nitrofuran-2-yl, 5-[1,3]-dioxolan-furan-2-yl, 5-Carbonsäure-furan-2-yl, Thien-2-yl, Thien-3-yl und 5-Carbonsäure-2-thiophen; oder COOR¹⁰ steht.

4. *C*-Imidazo[1,2-a]pyridin-3-yl-methylamin gemäß einem der Ansprüche 1 bis 3, **dadurch gekennzeichnet, daß** R¹ für C₂H₅, CH₃, CF₃ oder Cl, insbesondere für CH₃, CF₃ oder Cl, vorzugsweise für CH₃ steht.

5. *C*-ltnidazo[1,2-a]pyridin-3-yl-methylamin gemäß einem der Ansprüche 1 bis 3, **dadurch gekennzeichnet, daß** R⁵ für
H; C₁₋₆-Alkyl, ein- oder mehrfach substituiert mit F, Cl, Br, 1, NH₂, SH oder OH, oder unsubstituiert, gesättigt oder ungesättigt, verzweigt oder unverzweigt; oder Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert mit F, Ct, Br, I, NH₂, SH oder OH, oder unsubstituiert, verzweigt oder unverzweigt; steht.

6. Verwendung gemäß einem der Ansprüche 1 bis 5, **dadurch gekennzeichnet, daß R**^{**3**}
für H; C₁₋₆-Alkyl, ein- oder mehrfach substituiert mit F, Cl, Br, I, NH₂, SH oder OH, oder unsubstituiert, gesättigt oder ungesättigt, verzweigt oder unverzweigt; oder Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert mit F, Cl, Br, 1, NH₂, SH oder OH, oder unsubstituiert, verzweigt oder unverzweigt; steht.

7. *C*-lmidazo[1,2-a]pyridin-3-yl-methylamin gemäß einem der Ansprüche 1 bis 6, **dadurch gekennzeichnet, daß** R² für H steht

8. *C*-Imidazo[1,2-a]pyridin-3-yl-methylamin gemäß einem der Ansprüche 1 bis 7, **dadurch gekennzeichnet, daß** R⁶
für H; C₁₋₆-Alkyl, ein- oder mehrfach substituiert mit F, Cl, Br, I, NH₂, SH oder OH, oder unsubstituiert, gesättigt oder ungesättigt, verzweigt oder unverzweigt; Methyl, Ethyl, Methylethyl, 1,1-Dimethylethyl, Propyl, 2-Methylpropyl, 1-Methylpropyl, 1-Ethylpropyl, Butyl, i-Butyl, n-Butyl, tert.-Butyl, 1-Methylbutyl, 2-Methylbutyl, 3-Methylbutyl, 2,2-Dimethylbutyl, Pentyl, 2-Methylpentyl, 3-Methylpentyl, 1,1-Dimethylpropyl, 1,2-Dimethylpropyl, 2,2-Dimethylpropyl, 1-Ethylpropyl oder Hexyl, jeweils ein- oder mehrfach substituiert mit F, Cl, Br, I, NH₂, SH oder OH, oder unsubstituiert, verzweigt oder unverzweigt; steht.

9. C-Imidazo[1,2-a]pyridin-3-yl-methylamin gemäß einem der Ansprüche 1 bis 6, **dadurch gekennzeichnet, daß** die Reste R⁵ und R⁶ zusammen einen Ring bilden und CH₂CH₂OCH₂CH₂, CH₂CH₂NR¹¹CH₂CH₂ oder (CH₂) ₃₋₆ bedeuten.

10. Arzneimittel enthaltend mindestens ein C-Imidazo[1,2-a]pyridin-3-yl-methylamin gemäß einem der Ansprüche 1 bis 9 sowie gegebenenfalls Hilfsstoffe.

11. Verwendung einer Verbindung gemäß einem der Ansprüche 1 bis 9 zur Herstellung eines Arzneimittels zur Behandlung von Migräne, septischem Schock, neurodegenerativen Erkrankungen, vorzugsweise Multipler Sklerose, Morbus Parkinson, Morbus Alzheimer oder Morbus Huntington, Entzündungsschmerz, cerebraler ischämie, Diabetes, Meningitis, Arteriosklerose, Krebserkrankungen oder zur Wundheilung.

## Revendications

1. C-imidazo[1,2-a]-pyridine-3-yl-méthylamine substituée de formule générale I 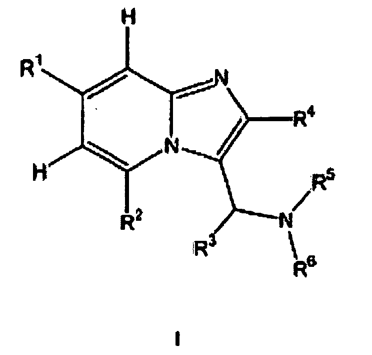 où dans chaque cas,
R¹ représente OH, SH, NH₂, NHCH₃, N(CH₃)₂, CH₃, CH₂Cl, CH₂F, CHF₂, CF₃, OCH₃, OCH₂Cl, OCH₂F, OCHF₂, OCF₃, SCH₃, SCF₃, C₂H₅, CHClCH₃, CH₂CH₂Cl, CHFCH₃, CH₂CH₂F, CH₂CHF₂, CH₂CF₃, OC₂H₅, COOH, CH₂OH, CHOHCH₃, CH₂CH₂OH, CN, NO₂, F, Br, I ou Cl,
R² représente H ou CH₃,
R³ représente H ; un reste alkyle en C₁ à C₆, substitué une ou plusieurs fois avec F, Cl, Br, I, NH₂, SH ou OH, ou non substitué, saturé ou non saturé, ramifié ou non ramifié ; un reste méthyle, éthyle, méthyléthyle, 1,1-diméthyléthyle, propyle, 2-méthylpropyle, 1-méthylpropyle, 1-éthylpropyle, butyle, isobutyle, n-butyle, tertiobutyle, 1-méthylbutyle, 2-méthylbutyle, 3-méthylbutyle, 2,2-diméthylbutyle, pentyle, 2-méthylpentyle, 3-méthylpentyle, 1,1-diméthylpropyle, 1,2-diméthylpropyle, 2,2-diméthylpropyle, 1-éthylpropyle ou hexyle, chacun substitué une ou plusieurs fois avec F, Cl, Br, I, NH₂, SH ou OH, ou non substitué, ramifié ou non ramifié ; un reste cycloalkyle en C₃ à C₈, dans chaque cas substitué une ou plusieurs fois avec F, Cl, Br, I, NH₂, SH ou OH, ou non substitué, insaturé une fois, ou saturé ; un reste cyclopropyle, cyclobutyle, cyclopentyle, cyclohexyle, cycloheptyle, cyclooctyle, 2-méthylcyclopropyle, cyclopropylméthyle, 2-méthylcyclobutyle, 3-méthylcyclobutyle, cyclobutylméthyle, chacun substitué avec CO₂H ou CO₂-éthyle, ou non substitué ; ou un reste phényle, naphtyle ou anthracényle, chacun substitué avec F, CF₃, OH ou OCH₃ ou non substitué ; ou un reste pyridine-2-yle, pyridine-3-yle, furanne-2-yle, furanne-3-yle, 5-hydroxyméthylène-furanne-2-yle, 5-nitrofuranne-2-yle, 5-[1,3]-dioxolane-furanne-2-yle, 5-carboxy-furanne-2-yle, thién-2-yle, thién-3-yle et 5-carboxy-2-thiophène ; ou COOR¹⁰,
R⁴ H ; un reste alkyle en C₁ à C₁₂, substitué une ou plusieurs fois avec F, Cl, Br, I, NH₂, SH ou OH, ou non substitué, saturé ou non saturé, ramifié ou non ramifié ; un reste méthyle, éthyle, méthyléthyle, 1,1-diméthyléthyle, propyle, 2-méthylpropyle, 1-méthyl-propyle, 1-éthylpropyle, butyle, isobutyle, n-butyle, tertiobutyle, 1-méthylbutyle, 2-méthylbutyle, 3-méthylbutyle, 2,2-diméthylbutyle, pentyle, 2-méthylpentyle, 3-méthylpentyle, 1,1-diméthylpropyle, 1,2-diméthylpropyle, 2,2-diméthylpropyle, 1-éthylpropyle ou hexyle, heptyle, octyle, nonyle, décyle, undécyle, dodécyle, chacun substitué une ou plusieurs fois avec F, Cl, Br, I, NH₂, SH ou OH, ou non substitué, ramifié ou non ramifié ; un reste cycloalkyle en C₃ à C₈, dans chaque cas substitué une ou plusieurs fois avec F, Cl, Br, I, NH₂, SH ou OH ou non substitué, insaturé une fois ou saturé ; un reste cyclopropyle, cyclobutyle, cyclopentyle, cyclohexyle, cycloheptyle, cyclooctyle, 2-méthylcyclopropyle, cyclopropylméthyle, 2-méthylcyclobutyle, 3-méthylcyclobutyle, cyclobutylméthyle, chacun substitué avec CO₂H ou CO₂-éthyle, ou non substitué ; ou un reste de formule II 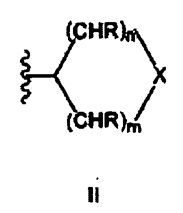
où dans chaque cas
n est un nombre compris entre 0 et 6,
m est un nombre compris entre 0 et 6,
1 ≤ m + n ≤ 6,
X représente O, S, SO, SO₂ ou NR⁷,
les R représentent, indépendamment l'un de l'autre, H, F, Br, I, Cl, OH, SH, NH₂, NO₂, CH₃, C₂H₅, OCH₃, OC₂H₅, OCF₃,
R⁵ représente H ; un reste alkyle en C₁ à C₆, substitué une ou plusieurs fois avec F, Cl, Br, I, NH₂, SH ou OH, ou non substitué, saturé ou non saturé, ramifié ou non ramifié ; un reste méthyle, éthyle, méthyléthyle, 1,1-diméthyléthyle, propyle, 2-méthylpropyle, 1-méthylpropyle, 1-éthylpropyle, butyle, isobutyle, n-butyle, tertiobutyle, 1-méthylbutyle, 2-méthylbutyle, 3-méthylbutyle, 2,2-diméthylbutyle, pentyle, 2-méthylpentyle, 3-méthylpentyle, 1,1-diméthylpropyle, 1,2-diméthylpropyle, 2,2-diméthylpropyle, 1-éthylpropyle ou hexyle, chacun substitué une ou plusieurs fois avec F, Cl, Br, I, NH₂, SH ou OH, ou non substitué, ramifié ou non ramifié ; ou un reste cycloalkyle en C₃ à C₈, dans chaque cas substitué une ou plusieurs fois avec F, Cl, Br, I, NH₂, SH ou OH, ou non substitué, insaturé une fois ou saturé ; un reste cyclopropyle, cyclobutyle, cyclopentyle, cyclohexyle, cycloheptyle, cyclooctyle, 2-méthylcyclopropyle, cyclopropylméthyle, 2-méthylcyclobutyle, 3-méthylcyclobutyle, cyclobutylméthyle, chacun substitué avec CO₂H ou CO₂-éthyle, ou non substitué ;
R⁶ représente H ; un reste alkyle en C₁ à C₆, substitué une ou plusieurs fois avec F, Cl, Br, I, NH₂, SH ou OH, ou non substitué, saturé ou non saturé, ramifié ou non ramifié ; un reste méthyle, éthyle, méthyléthyle, 1,1-diméthyléthyle, propyle, 2-méthylpropyle, 1-méthylpropyle, 1-éthylpropyle, butyle, isobutyle, n-butyle, tertiobutyle, 1-méthylbutyle, 2-méthylbutyle, 3-méthylbutyle, 2,2-diméthylbutyle, pentyle, 2-méthylpentyle, 3-méthylpentyle, 1,1-diméthylpropyle, 1,2-diméthylpropyle, 2,2-diméthylpropyle, 1-éthylpropyle ou hexyle, chacun substitué une ou plusieurs fois avec F, Cl, Br, I, NH₂, SH ou OH, ou non substitué, ramifié ou non ramifié ; ou un reste cycloalkyle en C₃ à C₈, dans chaque cas substitué une ou plusieurs fois avec F, Cl, Br, I, NH₂, SH ou OH, ou non substitué, insaturé une fois ou saturé ; un reste cyclopropyle, cyclobutyle, cyclopentyle, cyclohexyle, cycloheptyle, cyclooctyle, 2-méthylcyclopropyle, cyclopropylméthyle, 2-méthylcyclobutyle, 3-méthylcyclobutyle, cyclobutylméthyle, chacun substitué avec CO₂H ou CO₂-éthyle, ou non substitué ; ou un reste phényle, naphtyle ou anthracényle, chacun substitué avec F, CF₃, OH ou OCH₃ ou non substitué ; ou un reste pyridine-2-yle, pyridine-3-yle, furanne-2-yle, furanne-3-yle, 5-hydroxyméthylène-furanne-2-yle, 5-nitrofuranne-2-yle, 5-[1,3]-dioxolane-furanne-2-yle, 5-carboxy-furanne-2-yle, thién-2-yle, thién-3-yle et 5-carboxy-2-thiophène ;
ou bien les restes R⁵ et R⁶ forment ensemble un noyau et représentent CH₂CH₂OCH₂CH₂, CH₂CH₂NR¹¹CH₂CH₂ ou (CH₂)₃₋₆,
R⁷ représente H, un reste alkyle en C₁ à C₆, avantageusement méthyle, éthyle, propyle, méthyléthyle, butyle, 1-méthylpropyle, 2-méthylpropyle, 1,1-diméthyléthyle, pentyle, 1,1-diméthylpropyle, 1,2-diméthylpropyle, 2,2-diméthylpropyle, 1-éthylpropyle ou hexyle, un reste cycloalkyle en C₃ à C₈, avantageusement cyclopropyle, 2-méthylcyclopropyle, cyclopropylméthyle, cyclobutyle, 2-méthylcyclobutyle, 3-méthylcyclobutyle, cyclobutylméthyle, cyclopentyle, cyclohexyle ou cyclooctyle, un reste acyle C(O)R⁸ ou un reste sulfonyle S(O₂)R⁹,
R⁸ représente H, un reste alkyle en C₁ à C₆ ou un reste phényle, naphtyle ou anthracényle,
R⁹ représente H, un reste alkyle en C₁ à C₆ ou un reste phényle, naphtyle ou anthracényle,
R¹⁰ représente H, un reste alkyle en C₁ à C₆ ou un reste phényle, naphtyle ou anthracényle,
R¹¹ représente H, un reste alkyle en C₁ à C₆ ou cycloalkyle en C₃ à C₈ ou un reste phényle, naphtyle ou anthracényle ; ou un reste pyridine-2-yle, pyridine-3-yle, furanne-2-yle, furanne-3-yle, 5-hydroxyméthylène-furanne-2-yle, 5-nitrofuranne-2-yle, 5-[1,3]-dioxolane-furanne-2-yle, 5-carboxy-furanne-2yle, thién-2-yle, thién-3-yle et 5-carboxy-2-thiophène ; un reste phényle, naphtyle ou anthracényle, ou pyridine-2-yle, pyridine-3-yle, furanne-2-yle, furanne-3-yle, 5-hydroxyméthylène-furanne-2-yle, 5-nitro-furanne-2-yle, 5-[1,3]-dioxolane-furanne-2-yle, 5-carboxy-furanne-2-yle, thién-2-yle, thién-3-yle et 5-carboxy-2-thiophène en liaison par l'intermédiaire d'un groupe alkylène en C₁ à C₃ ; un reste acyle C(O)R¹² ou sulfonyle S(O₂)R¹³,
R¹² représente H, un reste alkyle en C₁ à C₆ ou un reste phényle, naphtyle ou anthracényle,
R¹³ représente H, un reste alkyle en C₁ à C₆ ou un reste phényle, naphtyle ou anthracényle,
le cas échéant sous forme de ses racémates, de ses stéréoisomères purs, en particulier de ses énantiomères ou diastéréoisomères, ou sous forme de mélanges des stéréoisomères, en particulier des énantiomères ou des diastéréoisomères, dans un rapport de mélange quelconque, sous la forme représentée ou sous forme de ses acides ou de ses bases ou sous forme de ses sels, en particulier des sels acceptables du point de vue physiologique, ou sous forme de ses produits de solvatation, en particulier des hydrates ;
sous réserve que les composés suivants : 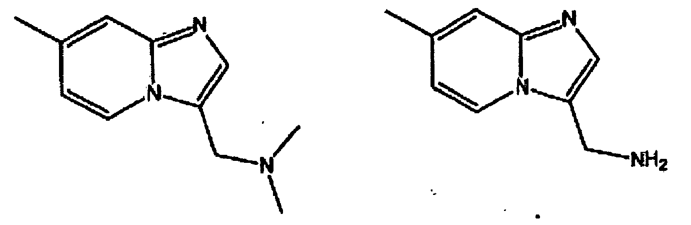 soient exclus de la protection.

2. C-imidazo[1,2-a]-pyridine-3-yl-méthylamine suivant la revendication 1, **caractérisée en ce que** R⁴ représente
un reste alkyle en C₁ à C₁₂, substitué une ou plusieurs fois avec F, Cl, Br, I, NH₂, SH ou OH, ou non substitué, saturé ou non saturé, ramifié ou non ramifié ; un reste méthyle, éthyle, méthyléthyle, 1,1-diméthyléthyle, propyle, 2-méthylpropyle, 1-méthylpropyle, 1-éthylpropyle, butyle, iso-butyle, n-butyle, tertiobutyle, 1-méthylbutyle, 2-méthylbutyle, 3-méthylbutyle, 2,2-diméthylbutyle, pentyle, 2-méthylpentyle, 3-méthylpentyle, 1,1-diméthylpropyle, 1,2-diméthylpropyle, 2,2-diméthylpropyle, 1-éthylpropyle ou hexyle, heptyle, octyle, nonyle, décyle, undécyle, dodécyle, chacun substitué une ou plusieurs fois avec F, Cl, Br, I, NH₂, SH ou OH, ou non substitué, ramifié ou non ramifié ; un reste cycloalkyle en C₃ à C₈, substitué dans chaque cas une ou plusieurs fois avec F, Cl, Br, I, NH₂, SH ou OH, ou non substitué, insaturé une fois ou saturé ; un reste cyclopropyle, cyclobutyle, cyclopentyle, cyclohexyle, cycloheptyle, cyclooctyle, 2-méthylcyclopropyle, cyclopropylméthyle, 2-méthylcyclobutyle, 3-méthylcyclobutyle, cyclobutylméthyle ; ou un reste de formule II 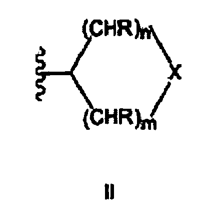 où dans chaque cas
n est un nombre compris entre 0 et 6,
m est un nombre compris entre 0 et 6, 1 ≤ m + n ≤ 6,
X représente 0, S, SO, SO₂ ou NR⁷,
les R représentent indépendamment l'un de l'autre H, F, Br, I, Cl, OH, SH, NH₂, NO₂, CH₃, C₂H₅ , OCH₃, OC₂H₅, OCF₃.

3. C-imidazo[1,2-a}-pyridine-3-yl-méthylamine suivant l'une des revendications 1 ou 2, **caractérisée en ce que** R³ représente
un reste alkyle en C₁ à C₆, substitué une ou plusieurs fois avec F, Cl, Br, I, NH₂, SH ou OH, ou non substitué, saturé ou non saturé, ramifié ou non ramifié ; un reste méthyle, éthyle, méthyléthyle, 1,1-diméthyléthyle, propyle, 2-méthylpropyle, 1-méthylpropyle, 1-éthylpropyle, butyle, isobutyle, n-butyle, tertiobutyle, 1-méthylbutyle, 2-méthylbutyle, 3-méthylbutyle, 2,2-diméthylbutyle, pentyle, 2-méthylpentyle, 3-méthylpentyle, 1,1-diméthylpropyle, 1,2-diméthylpropyle, 2,2-diméthylpropyle, 1-éthylpropyle ou hexyle, chacun substitué une ou plusieurs fois avec F, Cl, Br, I, NH₂, SH ou OH, ou non substitué, ramifié ou non ramifié ; un reste cycloalkyle en C₃ à C₈, substitué dans chaque cas une ou plusieurs fois avec F, Cl, Br, I, NH₂, SH ou OH, ou non substitué, insaturé une fois ou saturé ; un reste cyclopropyle, cyclobutyle, cyclopentyle, cyclohexyle, cycloheptyle, cyclooctyle, 2-méthylcyclopropyle, cyclopropylméthyle, 2-méthylcyclobutyle, 3-méthylcyclobutyle, cyclobutylméthyle ; ou un reste phényle, naphtyle ou anthracényle, chacun substitué avec F, CF₃, OH ou OCH₃ ou non substitué ; ou un reste pyridine-2-yle, pyridine-3-yle, furanne-2-yle, furanne-3-yle, 5-hydroxyméthylène-furanne-2-yle, 5-nitrofuranne-2-yle, 5-[1,3]-dioxolane-furanne-2-yle, 5-carboxy-furanne-2-yle, thién-2-yle, thién-3-yle et 5-carboxy-2-thiophène ; ou bien COOR¹⁰.

4. C-imidazo[1,2-a]-pyridine-3-yl-méthylamine suivant l'une des revendications 1 à 3, **caractérisée en ce que** R¹ représente C₂H₅, CH₃, CF₃ ou Cl, en particulier CH₃, CF₃ ou Cl, notamment CH₃.

5. C-imidazo[1,2-a]-pyridine-3-yl-méthylamine suivant l'une des revendications 1 à 3, **caractérisée en ce que** R⁵ représente
H ; un reste alkyle en C₁ à C₆, substitué une ou plusieurs fois avec F, Cl, Br, I, NH₂, SH ou OH, ou non substitué, saturé ou non saturé, ramifié ou non ramifié ; ou un reste méthyle, éthyle, méthyléthyle, 1,1-diméthyléthyle, propyle, 2-méthylpropyle, 1-méthylpropyle, 1-éthylpropyle, butyle, isobutyle, n-butyle, tertiobutyle, 1-méthylbutyle, 2-méthylbutyle, 3-méthylbutyle, 2,2-diméthylbutyle, pentyle, 2-méthylpentyle, 3-méthylpentyle, 1,1-diméthylpropyle, 1,2-diméthylpropyle, 2,2-diméthylpropyle, 1-éthylpropyle ou hexyle, chacun substitué une ou plusieurs fois avec F, Cl, Br, I, NH₂, SH ou OH, ou non substitué, ramifié ou non ramifié.

6. Utilisation suivant l'une des revendications 1 à 5, **caractérisée ce que** R³ représente
H ; un reste alkyle en C₁ à C₆, substitué une ou plusieurs fois avec F, Cl, Br, I, NH₂, SH ou OH, ou non substitué, saturé ou non saturé, ramifié ou non ramifié ; ou un reste méthyle, éthyle, méthyléthyle, 1,1-diméthyléthyle, propyle, 2-méthylpropyle, 1-méthylpropyle, 1-éthylpropyle, butyle, isobutyle, n-butyle, tertiobutyle, 1-méthylbutyle, 2-méthylbutyle, 3-méthylbutyle, 2,2-diméthylbutyle, pentyle, 2-méthylpentyle, 3-méthylpentyle, 1,1-diméthylpropyle, 1,2-diméthylpropyle, 2,2-diméthylpropyle, 1-éthylpropyle ou hexyle, chacun substitué une ou plusieurs fois avec F, Cl, Br, I, NH₂, SH ou OH, ou non substitué, ramifié ou non ramifié.

7. C-imidazo[1,2-a]-pyridine-3-yl-méthylamine suivant l'une des revendications 1 à 6, **caractérisée en ce que** R² représente H.

8. C-imidazo[1,2-a]-pyridine-3-yl-méthylamine suivant l'une des revendications 1 à 7, **caractérisée en ce que** R⁶ représente
H ; un reste alkyle en C₁ à C₆, substitué une ou plusieurs fois avec F, Cl, Br, I, NH₂, SH ou OH, ou non substitué, saturé ou non saturé, ramifié ou non ramifié ; un reste méthyle, éthyle, méthyléthyle, 1,1-diméthyléthyle, propyle, 2-méthylpropyle, 1-méthylpropyle, 1-éthylpropyle, butyle, isobutyle, n-butyle, tertiobutyle, 1-méthylbutyle, 2-méthylbutyle, 3-méthylbutyle, 2,2-diméthylbutyle, pentyle, 2-méthylpentyle, 3-méthylpentyle, 1,1-diméthylpropyle, 1,2-diméthylpropyle, 2,2-diméthylpropyle, 1-éthylpropyle ou hexyle, chacun substitué une ou plusieurs fois avec F, Cl, Br, I, NH₂, SH ou OH, ou non substitué, ramifié ou non ramifié.

9. C-imidazo[1,2-a]-pyridine-3-yl-méthylamine suivant l'une des revendications 1 à 6, **caractérisée en ce que** les restes R⁵ et R⁶ forment ensemble un noyau et désignent CH₂CH₂OCH₂CH₂, CH₂CH₂NR¹¹CH₂CH₂ ou (CH₂)₃₋₆.

10. Médicament contenant au moins une C-imidazo-[1,2-a]-pyridine-3-ylméthylamine suivant l'une des revendications 1 à 9 ainsi que, le cas échéant, des substances auxiliaires.

11. Utilisation d'un composé suivant l'une des revendications 1 à 9 pour la préparation d'un médicament destiné au traitement de la migraine, du choc septique, de maladies neurodégénératives, avantageusement de la sclérose en plaques, de la maladie de Parkinson, de la maladie d'Alzheimer ou de la maladie de Huntington, de la douleur liée à une inflammation, de l'ischémie cérébrale, du diabète, de la méningite, de l'artériosclérose, de maladies cancéreuses ou pour la cicatrisation.

## Claims

1. Substituted C-imidazo[1,2-α]pyridin-3-yl-methylamine of the general formula I, 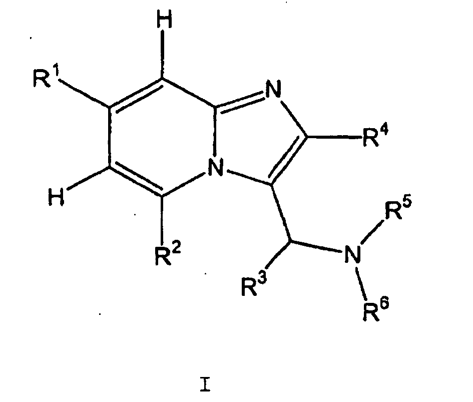 wherein in each case
R¹ denotes OH, SH, NH₂, NHCH₃, N(CH₃)₂, CH₃, CH₂Cl, CH₂F, CHF₂, CF₃, OCH₃, OCH₂Cl, OCH₂F, OCHF₂, OCF₃, SCH₃, SCF₃, C₂H₅, CHClCH₃, CH₂CH₂Cl, CHFCH₃, CH₂CH₂F, CH₂CHF₂, CH₂CF₃, OC₂H₅, COOH, CH₂OH, CHOHCH₃, CH₂CH₂OH, CN, NO₂, F, Br, I or Cl,
R² denotes H or CH₃,
R³ denotes H; C₁₋₆-alkyl, singly or multiply substituted with F, Cl, Br, I, NH₂, SH or OH, or unsubstituted, saturated or unsaturated, branched or unbranched; methyl, ethyl, methylethyl, 1,1-dimethylethyl, propyl, 2-methylpropyl, 1-methylpropyl, 1-ethylpropyl, butyl, i-butyl, n-butyl, tert.-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2- dimethylpropyl, 1-ethylpropyl or hexyl, in each case singly or multiply substituted with F, Cl, Br, I, NH₂, SH or OH, or unsubstituted, branched or unbranched; C₃₋₈-cycloalkyl, in each case singly or multiply substituted with F, Cl, Br, I, NH₂, SH or OH, or unsubstituted, singly unsaturated or saturated; cyclopropyl, cyclobutyl, cyclopentyl, cyclohexyl, cycloheptyl, cyclooctyl, 2-methylcyclopropyl, cyclopropylmethyl, 2-methylcyclobutyl, 3-methylcyclobutyl, cyclobutylmethyl, in each case substituted with CO₂H or CO₂ethyl,or unsubstituted; or phenyl, naphthyl or anthracenyl, in each case substituted with F, CF₃, OH or OCH₃ or unsubstituted; or pyridin-2-yl, pyridin-3-yl, furan-2-yl, furan-3-yl, 5-hydroxymethylene-furan-2-yl, 5-nitro-furan-2-yl, 5-[1,3]-dioxolane-furan-2-yl, 5-carboxylic acid-furan-2-yl, thien-2-yl, thien-3-yl and 5-carboxylic acid-2-thiophene; or COOR¹⁰,
R⁴ denotes H; C₁₋₁₂-alkyl, singly or multiply substituted with F, Cl, Br, I, NH₂, SH or OH, or unsubstituted, saturated or unsaturated, branched or unbranched; methyl, ethyl, methylethyl, 1,1-dimethylethyl, propyl, 2-methylpropyl, 1-methylpropyl, 1-ethylpropyl, butyl, i-butyl, n-butyl, tert.-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2-dimethylpropyl, 1-ethylpropyl or hexyl, heptyl, octyl, nonyl, decyl, undecyl, dodecyl, in each case singly or multiply substituted with F, Cl, Br, I, NH₂, SH or OH, or unsubstituted, branched or unbranched; C₃₋₈-cycloalkyl, in each case singly or multiply substituted with F, Cl, Br, I, NH₂, SH or OH, or unsubstituted, singly unsaturated or saturated; cyclopropyl, cyclobutyl, cyclopentyl, cyclohexyl, cycloheptyl, cyclooctyl, 2-methylcyclopropyl, cyclopropylmethyl, 2-methylcyclobutyl, 3-methylcyclobutyl, cyclobutylmethyl, in each case substituted with CO₂H or CO₂ethyl, or unsubstituted; or a radical according to formula II, 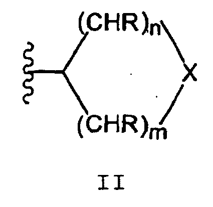
wherein in each case
n denotes a number between 0 and 6,
m denotes a number between 0 and 6,
1≤m+n≤6,
X denotes O, S, SO, SO₂ or NR⁷,
R independently of one another denotes H, F, Br, I, Cl, OH, SH, NH₂, NO₂, CH₃, C₂H₅, OCH₃, OC₂H₅, OCF₃,
R⁵ denotes H; C₁₋₆-alkyl, singly or multiply substituted with F, Cl, Br, I, NH₂, SH or OH, or unsubstituted, saturated or unsaturated, branched or unbranched; methyl, ethyl, methylethyl, 1,1-dimethylethyl, propyl, 2-methylpropyl, 1-methylpropyl, 1-ethylpropyl, butyl, i-butyl, n-butyl, tert.-butyl, 1-methylbutyl, 2- methylbutyl, 3-ethylbutyl, 2,2-dimethylbutyl, pentyl, 2-methylpentyl, 3-methylpentyl, 1,1-dimethylpropyl, 1,2-dimethylpropyl, 2,2-dimethylpropyl, 1-ethylpropyl or hexyl, in each case singly or multiply substituted with F, Cl, Br, I, NH₂, SH or OH, or unsubstituted, branched or unbranched; or C₃₋₈-cycloalkyl, in each case singly or multiply substituted with F, Cl, Br, I, NH₂, SH or OH, or unsubstituted, singly unsaturated or saturated; cyclopropyl, cyclobutyl, cyclopentyl, cyclohexyl, cycloheptyl, cyclooctyl, 2-methylcyclopropyl, cyclopropylmethyl, 2-methylcyclobutyl, 3-methylcyclobutyl, cyclobutylmethyl, in each case substituted with CO₂H or CO₂ethyl,or unsubstituted;
R⁶ denotes H; C₁₋₆-alkyl, singly or multiply substituted with F, Cl, Br, I, NH₂, SH or OH, or unsubstituted, saturated or unsaturated, branched or unbranched; methyl, ethyl, methylethyl, 1,1-dimethylethyl, propyl, 2-methylpropyl, 1-methylpropyl, 1-ethylpropyl, butyl, i-butyl, n-butyl, tert.-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2-dimethylpropyl, 1-ethylpropyl or hexyl, in each case singly or multiply substituted with F, Cl, Br, I, NH₂, SH or OH, or unsubstituted, branched or unbranched; or C₃₋₈-cycloalkyl, in each case singly or multiply substituted with F, Cl, Br, I, NH₂, SH or OH, or unsubstituted, singly unsaturated or saturated; cyclopropyl, cyclobutyl, cyclopentyl, cyclohexyl, cycloheptyl, cyclooctyl, 2-methylcyclopropyl, cyclopropylmethyl, 2-methylcyclobutyl, 3-methylcyclobutyl, cyclobutylmethyl, in each case substituted with CO₂H or CO₂ethyl, or unsubstituted; or phenyl, naphthyl or anthracenyl, in each case substituted with F, CF₃, OH or OCH₃ or unsubstituted; or pyridin-2-yl, pyridin-3-yl, furan-2-yl, furan-3-yl, 5-hydroxymethylene-furan-2-yl, 5-nitro-furan-2-yl, 5-[1,3]-dioxolane-furan-2-yl, 5-carboxylic acid-furan-2-yl, thien-2-yl, thien-3-yl and 5-carboxylic acid-2-thiophene;
or the radicals R⁵ and R⁶ together form a ring and denote
CH₂CH₂OCH₂CH₂, CH₂CH₂NR¹¹CH₂CH₂ or (CH₂)₃₋₆,
R⁷ denotes H, a C₁₋₆-alkyl radical, preferably methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, 1-ethylpropyl or hexyl, a C₃₋₈-cycloalkyl radical, preferably cyclopropyl, 2-methylcyclopropyl, cyclopropylmethyl, cyclobutyl, 2-methylcyclobutyl, 3-methylcyclobutyl, cyclobutylmethyl, cyclopentyl, cyclohexyl or cyclooctyl, an acyl radical C(O)R⁸ or a sulfonyl radical S(O₂)R⁹,
R⁸ denotes H, a C₁₋₆-alkyl radical or a phenyl, naphthyl or anthracenyl radical,
R⁹ denotes H, a C₁₋₆-alkyl radical or a phenyl, naphthyl or anthracenyl radical,
R¹⁰ denotes H, a C₁₋₆-alkyl radical or a phenyl, naphthyl or anthracenyl radical,
R¹¹ denotes H, C₁₋₆-alkyl or C₃₋₈-cycloalkyl, or a phenyl, naphthyl or anthracenyl radical; or pyridin-2-yl, pyridin-3-yl, furan-2-yl, furan-3-yl, 5-hydroxymethylene-furan-2-yl, 5-nitro-furan-2-yl, 5-[1,3]-dioxolane-furan-2-yl, 5-carboxylic acid-furan-2-yl, thien-2-yl, thien-3-yl and 5-carboxylic acid-2-thiophene; phenyl, naphthyl or anthracenyl bound via C₁₋₃ alkylene, or pyridin-2-yl, pyridin-3-yl, furan-2-yl, furan-3-yl, 5-hydroxymethylene-furan-2-yl, 5-nitro-furan-2-yl, 5-[1,3]-dioxolane-furan-2-yl, 5-carboxylic acid-furan-2-yl, thien-2-yl, thien-3-yl and 5-carboxylic acid-2-thiophene; acyl C(O)R¹² or sulfonyl S (O₂) R¹³,
R¹² denotes H, a C₁₋₆-alkyl radical or a phenyl, naphthyl or anthracenyl radical,
R¹³ denotes H, a C₁₋₆-alkyl radical or a phenyl, naphthyl or anthracenyl radical,
optionally in the form of their racemates, their pure stereoisomers, in particular enantiomers or diastereomers, or in the form of mixtures of the stereoisomers, in particular the enantiomers or diastereomers, in an arbitrary mixture ratio; in the prepared form or in the form of their acids or their bases or in the form of their salts, in particular physiologically compatible salts, or in the form of their solvates, in particular hydrates;
with the proviso that the following compounds: 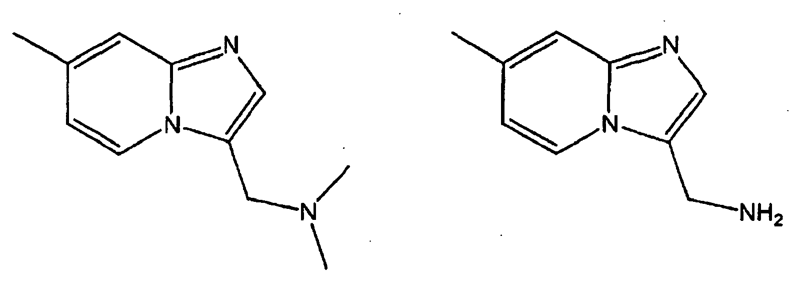 are excluded from protection.

2. C-imidazo[1,2-a]pyridin-3-yl-methylamine according to claim 1, **characterised in that** R⁴ denotes
C₁₋₁₂-alkyl, singly or multiply substituted with F, Cl, Br, I, NH₂, SH or OH, or unsubstituted, saturated or unsaturated, branched or unbranched; methyl, ethyl, methylethyl, 1,1-dimethylethyl, propyl, 2-methylpropyl, 1-methylpropyl, 1-ethylpropyl, butyl, i-butyl, n-butyl, tert.-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2-dimethylpropyl, 1-ethylpropyl or hexyl, heptyl, octyl, nonyl, decyl, undecyl, dodecyl, in each case singly or multiply substituted with F, Cl, Br, I, NH₂, SH or OH, or unsubstituted, branched or unbranched; C₃₋₈-cycloalkyl, in each case singly or multiply substituted with F, Cl, Br, I, NH₂, SH or OH, or unsubstituted, singly unsaturated or saturated; cyclopropyl, cyclobutyl, cyclopentyl, cyclohexyl, cycloheptyl, cyclooctyl, 2-methylcyclopropyl, cyclopropylmethyl, 2-methylcyclobutyl, 3-methylcyclobutyl, cyclobutylmethyl, or a radical according to formula II 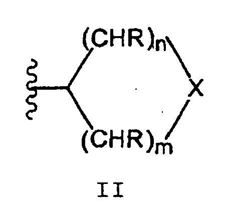 wherein in each case
n denotes a number between 0 and 6,
m denotes a number between 0 and 6,
1≤m+n≤6,
X denotes O, S, SO, SO or NR⁷,
R independently of one another denotes H, F, Br, I, C1, OH, SH, NH₂, NO₂, CH₃, C₂H₅, OCH₃, OC₂H₅, OCF₃.

3. C-imidazo[1,2-α]pyridin-3-yl-methylamine according to one of claims 1 and 2, **characterised in that** R³ denotes
C₁₋₆-alkyl, singly or multiply substituted with F, Cl, Br, I, NH₂, SH or OH, or unsubstituted, saturated or unsaturated, branched or unbranched; methyl, ethyl, methylethyl, 1,1-dimethylethyl, propyl, 2-methylpropyl, 1-methylpropyl, 1-ethylpropyl, butyl, i-butyl, n-butyl, tert.-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2-dimethylpropyl, 1-ethylpropyl or hexyl, in each case singly or multiply substituted with F, C1, Br, I, NH₂, SH or OH, or unsubstituted, branched or unbranched; C₃₋₈-cycloalkyl, in each case singly or multiply substituted with F, Cl, Br, I, NH₂, SH or OH, or unsubstituted, singly unsaturated or saturated; cyclopropyl, cyclobutyl, cyclopentyl, cyclohexyl, cycloheptyl, cyclooctyl, 2-methylcyclopropyl, cyclopropylmethyl, 2-methylcyclobutyl, 3-methylcyclobutyl, cyclobutylmethyl; or phenyl, naphthyl or anthracenyl, in each case substituted with F, CF₃, OH or OCH₃ or unsubstituted; or pyridin-2-yl, pyridin-3-yl, furan-2-yl, furan-3-yl, 5-hydroxymethylene-furan-2-yl, 5-nitro-furan-2-yl, 5-[1,3]-dioxolane-furan-2-yl, 5-carboxylic acid-furan-2-yl, thien-2-yl, thien-3-yl and 5-carboxylic acid-2-thiophene; or COOR¹⁰.

4. C-imidazo[1,2-α]pyridin-3-yl-methylamine according to one of claims 1 to 3, **characterised in that** R¹ denotes C₂H₅, CH₃, CF₃ or Cl, in particular CH₃, CF₃ or Cl, preferably CH₃.

5. C-imidazo[1,2-α]pyridin-3-yl-methylamine according to one of claims 1 to 3, **characterised in that** R⁵ denotes H; C₁-₆-alkyl, singly or multiply substituted with F, Cl, Br, I, NH₂, SH or OH, or unsubstituted, saturated or unsaturated, branched or unbranched; or methyl, ethyl, methylethyl, 1,1-dimethylethyl, propyl, 2-methylpropyl, 1-methylpropyl, 1-ethylpropyl, butyl, i-butyl, n-butyl, tert.-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2-dimethylpropyl, 1-ethylpropyl or hexyl, in each case singly or multiply substituted with F, Cl, Br, I, NH₂, SH or OH, or unsubstituted, branched or unbranched.

6. Use according to one of claims 1 to 5, **characterised in that** R³ denotes
H; C₁-₆-alkyl, singly or multiply substituted with F, Cl, Br, I, NH₂, SH or OH, or unsubstituted, saturated or unsaturated, branched or unbranched; or methyl, ethyl, methylethyl, 1,1-dimethylethyl, propyl, 2-methylpropyl, 1-methylpropyl, 1-ethylpropyl, butyl, i-butyl, n-butyl, tert.-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2-dimethylpropyl, 1-ethylpropyl or hexyl, in each case singly or multiply substituted with F, Cl, Br, I, NH₂, SH or OH, or unsubstituted, branched or unbranched.

7. C-imidazo[1,2-α]pyridin-3-yl-methylamine according to one of claims 1 to 6, **characterised in that** R² denotes H.

8. C-imidazo[1,2-α]pyridin-3-yl-methylamine according to one of claims 1 to 7, **characterised in that** R⁶ denotes
H; C₁₋₆-alkyl, singly or multiply substituted with F, Cl, Br, I, NH₂, SH or OH, or unsubstituted, saturated or unsaturated, branched or unbranched; methyl, ethyl, methylethyl, 1,1-dimethylethyl, propyl, 2-methylpropyl, 1-methylpropyl, 1-ethylpropyl, butyl, i-butyl, n-butyl, tert.-butyl, 1-methylbutyl, 2-methylbutyl, 3-methylbutyl, 2,2-dimethylbutyl, pentyl, 2-methylpentyl, 3-methylpentyl, 1,1-dimethylpropyl, 1,2-dimethylpropyl, 2,2-dimethylpropyl, 1-ethylpropyl or hexyl, in each case singly or multiply substituted with F, Cl, Br, I, NH₂, SH or OH, or unsubstituted, branched or unbranched.

9. C-imidazo[1,2-a]pyridin-3-yl-methylamine according to one of claims 1 to 6, **characterised in that** the radicals R⁵ and R⁶ together form a ring and denote CH₂CH₂OCH₂CH₂, CH₂CH₂NR¹¹CH₂CH₂ or (CH₂)₃₋₆.

10. Medicament containing at least one C-imidazo[1,2-α]pyridin-3-yl-methylamine according to one of claims 1 to 9 as well as optionally auxiliary substances.

11. Use of a compound according to one of claims 1 to 9 for the production of a medicament for treating migraine, septic shock, neurodegenerative diseases preferably multiple sclerosis, Parkinson's disease, Alzheimer's disease or Huntington's disease, inflammation pain, cerebral ischaemia, diabetes, meningitis, arterioscelorsis, cancers or for wound healing.
